# EUROPEAN PATENT APPLICATION

(11) **EP 2 018 861 A1**
(43) Date of publication of application: **28.01.2009**
(21) Application number: 07384031.6
(22) Date of filing: 26.07.2007
(51) Int. Cl.: A61K 31/404, A61K 31/4045, A61K 31/416, A61K 31/422, A61K 31/429, A61K 31/433, A61K 31/4439, A61K 31/454, A61K 31/4709, A61K 31/496, A61K 31/5377, A61P 3/04

(54) **5HT6-Ligands such as sulfonamide derivatives in drug-induced weight-gain**

(71) Applicant: Laboratorios del Dr. Esteve S.A., 08041 Barcelona (ES)
(72) Inventor: Fisas-Escasany, Maria Angeles, 08025 Barcelona (ES); Buschmann, Dr. Helmut D., 08960 Sant Just Desvern (DE)
(74) Representative: Peters, Hajo

(57) **Abstract**

The present invention relates to the use of Compounds binding to the 5HT6 receptor such as sulfonamide compounds of formula I in the manufacture of a medicament for the treatment of weight gain, obesity or associated cardio-metabolic risk factors induced by a therapeutic drug. 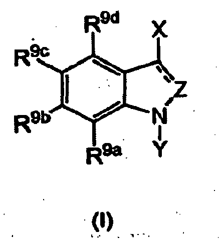 **wherein**
**--Z** either represents =C(R¹⁰) or -CH₂ or =N;
**and wherein**
**either**
**Y** represents -S(O₂)-R¹³, while X represents R¹;
**or**
**X** represents R¹, while **Y** represents (CH₂)ₙ-R¹¹ or
**Y** represents R¹, while **X** represents (CH₂)ₙ-R¹²;
while
one of R^{9a}, R^{9b}, R^{9c}, or R^{9d} represents N(R³)-S(O₂)-A, or N-(S(O₂)-A)₂.

## Description

The present invention relates to the use of Compounds binding to the 5HT6 receptor in the manufacture of a medicament for the treatment of weight gain, obesity or or associated cardio-metabolic risk factors induced by a therapeutic drug.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is a serotonin receptor identified by molecular cloning both in rats [F.J. Monsma, et al., Mol. Pharmacol., 1993, 43, 320; M. Ruat, et al., Biochem. Biophys. Res. Commun., 1993, 193, 268] and in humans [R. Kohen, et al., J. Neurochem., 1996, 66, 47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NY Acad. Sci., 1998, 861, 244; A. Bourson, et al., Br. J. Pharmacol. , 1998, 125, 1562; D.C. Rogers, et al., Br. J. Pharmacol. Suppl., 1999, 127, 22P; A. Bourson, et al., J. Pharmacol. Exp. Ther. , 1995, 274, 173; A.J. Sleight, et al., Behav. Brain Res. , 1996, 73, 245; T.A. Branchek, et al., nnu. Rev. Pharmacol. Toxicol. , 2000, 40, 319; C. Routledge, et al., Br. J. Pharmacol. , 2000, 130, 1606]. It has been shown that typical and atypical antipsychotic drugs for treating schizophrenia have a high affinity for 5-HT₆ receptors [B.L. Roth, et al., J. Pharmacol. Exp. Ther. , 1994, 268, 1403; C.E. Glatt, et al., Mol. Med. , 1995, 1, 398; F.J. Mosma, et al., Mol. Pharmacol. , 1993, 43, 320; T. Shinkai, et al., Am. J. med. Genet. , 1999, 88, 120]. Compounds with 5-HT₆ receptor affinity are useful for treating infant hyperkinesia (ADHD, attention deficit / hyperactivity disorder) [W.D. Hirst, et al., Br. J. Pharmacol. , 2000, 130, 1597; C. Gérard, et al., Brain Research , 1997, 746, 207; M.R. Pranzatelli, Drugs of Today , 1997, 33, 379]. Moreover, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases. One possible example of a food ingestion disorder is the weight gain that results from the intake of drugs called "drug-induced weight-gain" herein, whereby one of the possible examples include antipsychotic drugs. As stated in the reviews by Newcomer (2005) and Melkersson & Dahl (2004), the "second generation" or "atypical antipsychotic" drugs are known to cause serious weight-gain in approximately 50% of patients on therapy. In some cases, the increased adiposity has a deleterious effect on their cardio-metabolic status by causing dyslipidaemia, insulin resistance, impaired glucose tolerance, Type 2 diabetes and the Metabolic Syndrome as defined for example by the World Health Organisation (1999), American Heart Association National Cholesterol Education Program Adult Treatment Panel III (2002) and International Diabetes Federation (2005). The two atypical antipsychotic drugs with the greatest propensity to cause weight-gain and obesity are clozapine and olanzapine (see Newcomer, 2005; Melkersson & Dahl, 2004). Olanzapine treatment causes a mean increase in body weight of -5 kg, but the effect can be much greater in some individuals, especially children and adolescents (Haapasalo-Pesu & Saarijarvi, 2001; Ratzoni et al, 2002), and in addition, treatment of psychiatric disorders with this antipsychotic has also been reported to cause dyslipidaemia and Type 2 diabetes (see Newcomer, 2005; Melkersson & Dahl, 2004).

Thus, the object of the present invention was to find means for the treatment of drug-induced weight gain and its related risk factors.

Said object has been achieved by providing the use of a compound binding to the 5HT6-receptor in the manufacture of a medicament for the treatment of weight-gain, obesity and associated cardio-metabolic risk factors induced by a therapeutic drug.

It has now been surprisingly demonstrated that compounds binding to the 5HT6-receptor were able to prevent drug-induced weight-gain and obesity. Besides that the compounds, whose use is claimed in this invention, surprisingly also cause significant weight-loss below vehicle-treated control values. Such weight-loss thereby provides an unexpected clinical benefit to a patient, who is in need of therapy with a medication that causes weight-gain, and is overweight or obese and/or metabolically compromised, and as a consequence, is at increased cardio-metabolic risk. For this patient, the additional weight-loss and reduced adiposity provided by the compound/s binding to the 5HT6-receptor, whose use is claimed in this invention, will improve his/her glycaemic and/or plasma lipid profiles and greatly reduce his/her exposure to the risk of developing dyslipidaemia, Type 2 diabetes, developing Metabolic Syndrome, or suffering a cardio- or cerebrovascular accident.

"Compound/s binding to the 5HT6-receptor" (with "5HT6-Ligand" being also used in this description and being one and the same as "Compound/s binding to the 5HT6-receptor") as used in this application is/are defined as having an IC₅₀ value of ≤ 5000 nM, more preferably ≤ 1000 nM, more preferably ≤ 500 nM. More preferably, the IC₅₀ value is ≤ 250 nM. More preferably, the IC₅₀ value is ≤ 100 nM. Most preferably, the IC₅₀ value is ≤ 50 nM. Compound binding to the 5HT6-receptor may be partial agonists, full antagonists, full agonists, mixed agonist/antagonist or inverse agonists. Pharmacological test systems to determine all of these functionalities are well-known in the art. Thus in a preferred embodiment the "compound binding to the 5HT6-receptor" is a partial agonist. In another preferred embodiment the "compound/s binding to the 5HT6-receptor" is a full agonist. In another preferred embodiment the "compound binding to the 5HT6-receptor" is a full antagonist. In another preferred embodiment the "compound binding to the 5HT6-receptor" is a inverse agonist. In another preferred embodiment the "compound binding to the 5HT6-receptor" is a mixed agonist/antagonist.

"Treatment" as used in this application is defined as the treatment of a disease or of a medically relevant symptom, namely weight-gain, obesity or the increase of the associated cardio-metabolic risk factors, but also includes the prevention of the symptom or preventive activity during the development of the symptom.

"Therapeutic drug" as used in this application is defined as a drug or active substance, medical formulation or medicament being used in the therapeutic treatment of a mammal or patient. Specifically "therapeutic drug" refers to a drug or an active substance or pharmaceutical formulation or medicament being used in the treatment of a mammal or patient whose use has in all or only in specific cases of its use in mammals or a patient induced or caused weight-gain, obesity or the increase of the thus associated cardio-metabolic risk factors. Thus preferably the weight-gain, obesity or the increase of the thus associated cardio-metabolic risk factors is a known side-effect of the "therapeutic drug".

"Associated cardio-metabolic risk factors" as used in this application are defined as medically relevant factors or parameters, which alone or in combination with others increase the risk of developing cardio-, metabolic- or cardio-metabolic-diseases, or of suffering from a cardio- or cerebrovascular accident. Risk factors include the development of dyslipidemia, Metabolic syndrome, insulin resistance, impaired glucose tolerance or Type 2 diabetes.

Aliphatic radicals/groups, as referred to in the present invention, are optionally mono- or polysubstituted and may be branched or unbranched, saturated or unsaturated. Unsaturated aliphatic groups, as defined in the present invention, include alkenyl and alkinyl radicals. Saturated aliphatic groups, as defined in the present invention, include alkyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl.

In the context of this invention, alkyl radical or group is understood as meaning saturated, linear or branched hydrocarbons, which can be unsubstituted or mono- or polysubstituted. Alkenyl and alkinyl groups, on the other hand include groups like e.g. -CH=CH-CH₃ or -C≡C-CH₃, while the saturated alkyl encompasses e.g. -CH₃ and -CH₂-CH₃. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In the context of this invention cycloalkyl radical or group is understood as meaning saturated and unsaturated (but not aromatic) cyclic hydrocarbons (without a heteroatom in the ring), which can be unsubstituted or mono- or polysubstituted. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. However, mono- or polyunsaturated, preferably monounsaturated, cycloalkyls also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantly. In the context of this invention alkyl-cycloalkyl is understood as meaning a cycloalkyl group (see above) being connected to another atom through a C₁₋₆-alkyl group (see above), whereas the C₁₋₆-alkyl-group is always saturated and unsubstituted, and linear or branched.

In connection with alkyl or aliphatic group - unless defined otherwise - the term substituted in the context of this invention is understood as meaning replacement of at least one hydrogen radical by F, Cl, Br, I, NH₂, SH or OH, "polysubstituted" (more than once substituted) radicals being understood as meaning that the replacement takes effect both on different and on the same atoms several times with the same or different substituents, for example three times on the same C atom, as in the case of CF₃, or at different places, as in the case of e.g. - CH(OH)-CH=CH-CHCl₂. "Optionally at least monosubstituted" means either "monosubstituted", "polysubstituted" or - if the option is not fulfilled - "unsubstituted".

The term (CH₂)₃₋₆ is to be understood as meaning -CH₂-CH₂-CH₂-, -CH₂-CH₂-CH₂-CH₂-, - CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc.

The term "ring system" including "mono- or bicyclic ring system" according to the present invention refers to a ring sytem or ring sytems which comprises or comprise saturated, unsaturated or aromatic carbocyclic ring sytems which contain optionally at least one heteroatom as ring member and which are optionally at least mono-substituted. Said ring systems may be condensed to other carbocyclic ring systems such as aryl groups, naphtyl groups, heteroaryl groups, cycloalkyl groups, etc. Preferably the "ring system" may consist of 1 ring (monocyclic), or 2 (bicyclic) or 3 (tricyclic) rings being condensed.

An aryl radical or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention alkyl-aryl is understood as meaning an aryl group (see above) being connected to another atom through a C₁₋₆-alkyl-group (see above), whereas the C₁₋₆-alkyl-group is always saturated and unsubstituted, and linear or branched.

A heterocyclyl radical or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In the context of this invention alkyl-heterocylyl is understood as meaning a heterocyclyl group (see above) being connected to another atom through a C₁₋₆-alkyl group (see above), whereas the C₁₋₆-alkyl-group is always saturated and unsubstituted, and linear or branched.

In connection with aryl or alkyl-aryl, cycloalkyl or alkyl-cycloalkyl, heterocyclyl or alkyl-heterocyclyl, substituted is understood - unless defined otherwise - as meaning substitution of the ring-system of the aryl or alkyl-aryl, cycloalkyl or alkyl-cycloalkyl; heterocyclyl or alkyl-heterocyclyl by OH, SH, =O, halogen (F, Cl, Br, I), CN, NO₂, COOH; NRₓR_{y}, with Rₓ and R_{y} independently being either H or a saturated or unsaturated, linear or branched, substituted or unsubstituted C₁₋₆-alkyl; a saturated or unsaturated, linear or branched, substituted or unsubstituted C₁₋₆-alkyl; a saturated or unsaturated, linear or branched, substituted or unsubstituted -O-C₁₋₆-alkyl (alkoxy); a saturated or unsaturated, linear or branched, substituted or unsubstituted -S-C₁₋₆-alkyl; a saturated or unsaturated, linear or branched, substituted or unsubstituted -C(O)-C₁₋₆-alkyl-group; a saturated or unsaturated, linear or branched, substituted or unsubstituted -C(O)-O-C₁₋₆-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl. "Optionally at least monosubstituted" means either "monosubstituted", "polysubstituted" or - if the option is not fulfilled - "unsubstituted".

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids and in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Any compound that is a prodrug of a compound of formula (I) is within the scope of the invention. The term "prodrug" is used in its broadest sense and encompasses those Derivatives that are converted in vivo to the compounds of the invention. Such Derivatives would readily occur to those skilled in the art, and include, depending on the functional groups present in the molecule and without limitation, the following. Derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

In a preferred embodiment of the invention the compound binding to the 5HT6-receptor being used according to the invention is selected from sulfonamide compounds according to formula I, 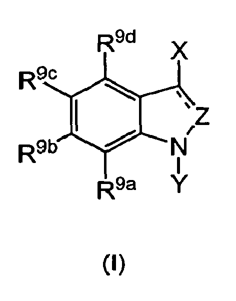 **wherein**
**--Z** either represents =C(R¹⁰) or -CH₂ or =N;
   **and wherein**
   **either**
**Y** represents -S(O₂)-R¹³, while X represents R¹;
   **or**
**X** represents R¹, while **Y** represents (CH₂)ₙ-R¹¹ or
**Y** represents R¹, while **X** represents (CH₂)ₙ-R¹²;
while
one of R^{9a}, R^{9b}, R^{9c}, or R^{9d} represents N(R³)-S(O₂)-A, or N-(S(O₂)-A)₂;
**and wherein**
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH,-C(=O)-C₁₋₅-alkyl, C(=O)-O-C₁₋₅-alkyl, -O-C(=O)-C₁₋₅-alkyl, oxo (=O), F, Cl, Br, I, -CN,-OCF₃, -SCF₃, -OH, -SH, -NH₂, -NH(C₁₋₅-alkyl), -N(C₁₋₅-alkyl)₂, -NH-C(=O)-C₁₋₅-alkyl,-N(C₁₋₅-alkyl)-C(=O)-C₁₋₅-alkyl, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy, benzyl, and pyridinyl and which may be condensed with a saturated or unsaturated mono- or bicyclic ring system, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S),-C(=O)-OH, -C(=O)-O-C₁₋₅-alkyl, -O-C(=O)-C₁₋₅-alkyl, F, Cl, Br, I, -CN, -CF₃, -OCF₃,-SCF₃, -OH, -SH, -NH₂, -NH(C₁₋₅-alkyl), -N(C₁₋₅-alkyl)₂, -NO₂, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, -OH,-NH₂, -SH, -O-CH₃, -O-C₂H₅, -NO₂, -CN, -NH-CH₃ and -S-CH₃ and wherein the heteroaryl radical contains 1, 2 or 3 heteroatom(s) independently selected from the group consisting of nitrogen, oxygen and sulfur as ring member(s);
- **R¹**: represents hydrogen, a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN,-NH-CH₃ and -S-CH₃; or an optionally at least monosubstituted alkyl-aryl radical;
- **R³**: represents a hydrogen atom; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅,-NO₂, -CN, -NH-CH₃ and -S-CH₃;
- **R^{9a}, R^{9b}, R^{9c}, R^{9d}** -: if not N(R³)-S(O₂)-A or N-(S(O₂)-A)₂- independently from one another, each represent a hydrogen atom; -NO₂; -NH₂; -SH; -OH; -CN; -C(=O)-H; -C(=O)-R^{'};-C(=O)-O-R'; -OR^{'}; -SR^{'}; -N(R^{'})-S(=O)₂-R^{"}; -NH-R^{'}; -NR^{*}R^{**}; F; Cl, Br; I; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl,-O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, -NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl, -NO₂, -CHO, -CF₂H, -CFH₂, -C(=O)-NH₂,-C(=O)-NH(C₁₋₅-alkyl), -C(=O)-N(C₁₋₅-alkyl)2, -S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene group and wherein the heteroaryl radical contains 1, 2 or 3 heteroatom(s) independently selected from the group consisting of nitrogen, oxygen and sulfur as ring member(s);
- **R¹⁰**: represents a hydrogen atom; -NO₂; -NH₂; -SH; -OH; -CN; -C(=O)-OH; -O-R'; -S-R';-C(=O)-OR"; a halogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl,-O-C(=O)-C₁₋₅-alkyl, F, Cl, Br, I, -CN, -CF₃, -OCF₃, -SCF₃, -OH, -SH, -NH₂,-NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5-, 6- or 7-membered and may contain 1, 2 or 3 heteroatom(s) as ring member(s) independently selected from the group consisting of nitrogen, oxygen and sulfur;
- **R¹²**: represents R¹¹,
or
represents 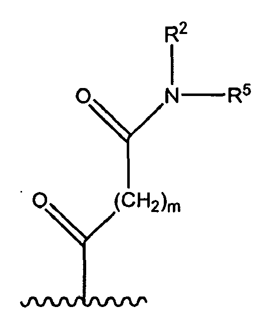 with n being 0;
or represents -C(OC₁₋₄₅-alkyl)-(CH₂)ₘ-R¹¹;
- **R¹³**: represents a saturated or unsaturated, optionally at least mono-substituted cycloaliphatic radical, or -CHR'R";
- **n**: being 0, 1, 2, 3 or 4;
- **m**: being 0, 1, 2, 3 or 4;
- **R'** and **R"**: identical or different, each represents a saturated or unsaturated, linear or branched, optionally at least mono-substituted aliphatic radical;
- **R*** and **R****: identical or different, each represents a saturated or unsaturated, linear or branched, optionally at least mono-substituted aliphatic radical;
or
- **R*** and **R****: together with the connecting nitrogen form an optionally at least monosubstituted heterocyclyl radical
- **R²**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅,-NO₂, -CN, -NH-CH₃ and -S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅,-NO₂, -CN, -NH-CH₃ and -S-CH₃;
or
- **R²** and **R⁵**: together with the bridging nitrogen form a saturated, unsaturated or aromatic 3- to 9-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl; -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂,-NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH,-C(=O)-O-C₁₋₅-alkyl, -O-C(=O)-C₁₋₅-alkyl, F, Cl, Br, I, -CN, -CF₃, -OCF₃, -SCF₃, -OH,-SH, -NH₂, -NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In one possible embodiment of the invention the proviso applies that the compound 5-Chloronaphthalene-2-sulfonic acid [3-(2-dimethylamino-ethyl)-1H-indol-5-yl]-amide is disclaimed from the use according to the invention.

In a preferred embodiment of the invention the compound binding to the 5HT6-receptor being used according to the invention according to formula I is selected from sulfonamide compounds according to formula la, 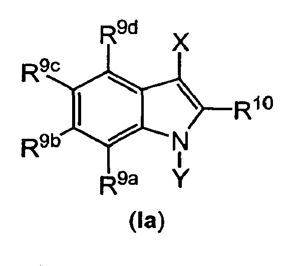 **wherein**
**either**
Y represents S(O₂)-R¹³, while X represents R¹;
   **or**
X represents R¹, while Y represents (CH₂)ₙ-R¹¹ or
Y represents R¹, while X represents (CH₂)ₙ-R¹²;
   while
one of R^{9a}, R^{9b}, R^{9c}, or R^{9d} represents N(R³)-S(O₂)-A,
**and wherein**
A, R¹_{;} R³, R^{9a}, R^{9b}, R^{9c}, R^{9d}, R¹⁰, R¹¹, R¹², R¹³ and n are as defined above.

In a preferred embodiment of the invention the compound binding to the 5HT6-receptor being used according to the invention is selected from sulfonamide compounds according to formula I, or la
**wherein**
**--Z** either represents =C(R¹⁰) or -CH₂ or =N;
   **and wherein**
   **either**
**Y** represents -S(O₂)-R¹³, while X represents R¹;
   **or**
**X** represents R¹, while **Y** represents (CH₂)ₙ-R¹¹ or
**Y** represents R¹, while **X** represents (CH₂)ₙ-R¹²;
   while
   one of R^{9a}, R^{9b}, R^{9c}, or R^{9d} represents N(R³)-S(O₂)-A, or N-(S(O₂)-A)₂;
**and wherein**
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;
- **R^{9a}, R^{9b}, R^{9c}, R^{9d}** -: if not N(R³)-S(O₂)-A or N-(S(O₂)-A)₂- independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl; -O-C₁₋₅-alkyl^{'}; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH;
- **R¹⁰**: represents a hydrogen atom;; a linear or branched optionally at least mono-substituted C₁₋₅-alkyl radical;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
- **R¹²**: represents R¹¹,
or
represents 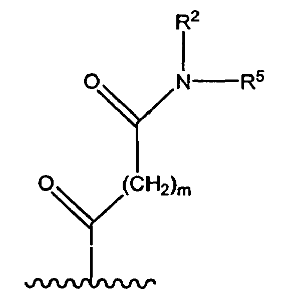 with n being 0;
or represents -C(OC₁₋₅-alkyl)-(CH₂)ₘ-R¹¹;
- **R¹³**: represents a saturated or unsaturated, optionally at least mono-substituted C₅₋₇-cycloaliphatic radical, or -CHR'R"; with **R'** and **R"** identical or different, each representing a saturated or unsaturated, linear or branched, optionally at least mono-substituted C₁₋₅-alkyl radical;
- **n**: being 0, 1, 2, 3 or 4;
- **m**: being 0, 1, 2, 3 or 4;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur.

In another preferred embodiment of the invention the compound binding to the 5HT6-receptor being used according to the invention according to formula I is selected from sulfonamide compounds according to formula Ib, or Ic, 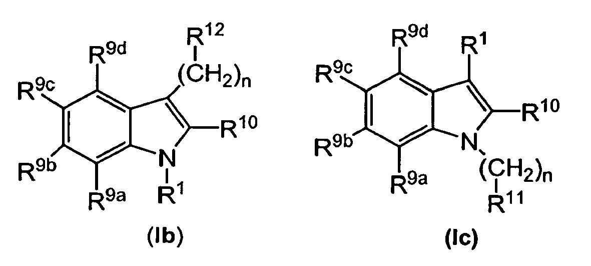 and wherein R¹, R^{9a}, R^{9b}, R^{9c}, R^{9d}, R¹⁰, R¹¹, R¹² and n have the meaning given above.

In another preferred embodiment of the invention the compound binding to the 5HT6-receptor being used according to the invention according to formula (Ib) is a compound according to formulas (Iba), (Ibb) or (Ibc), 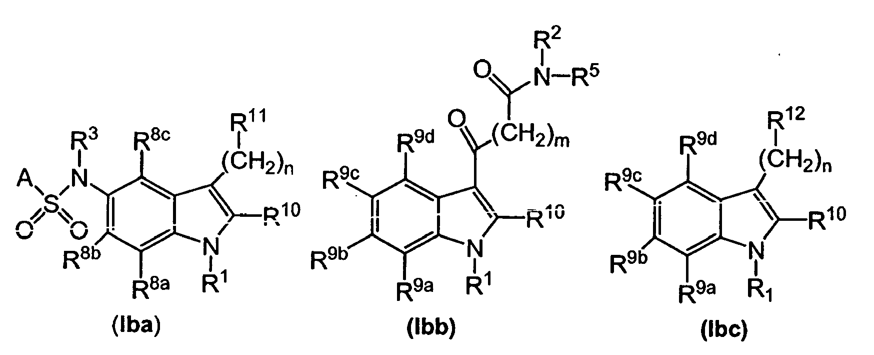 and wherein A, R¹, R², R³, R⁵, R^{9a}, R^{9b}, R^{9c}, R^{9d}, R¹⁰, R¹¹, R¹², m and n have the meaning given above and
wherein
- **R^{8a}, R^{8b}, R^{8c}**: independently from one another, each represent a hydrogen atom; -NO₂;-NH₂; -SH; -OH; -CN; -C(=O)-H; -C(=O)-R^{'}; -C(=O)-O-R'; -OR^{'}; -SR^{'}; -N(R^{'})-S(=O)₂-R^{"};-NH-R^{'}; -NR^{*}R^{**}; F; Cl, Br; I; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅,-NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl. -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, -NH₂,-NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl,-NO₂, -CHO, -CF₂H, -CFH₂, -C(=O)-NH₂, -C(=O)-NH(C₁₋₅-alkyl), -C(=O)-N(C₁₋₅-alkyl)2,-S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene group and wherein the heteroaryl radical contains 1, 2 or 3 heteroatom(s) independently selected from the group consisting of nitrogen, oxygen and sulfur as ring member(s);
preferably **R^{8a}, R^{8b}, R^{8c}** independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl;-O=C₁₋₅-alkyl; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH.

In another preferred embodiment of the invention the compound binding to the 5HT6-receptor being used according to the invention according to formula (Ic) is a compound according to formulas (Ica), (Icb), (Icc), or (Icd), 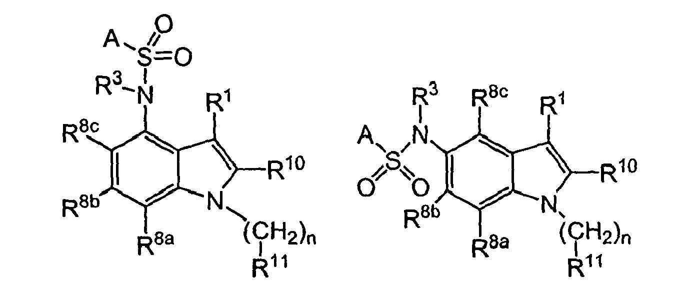 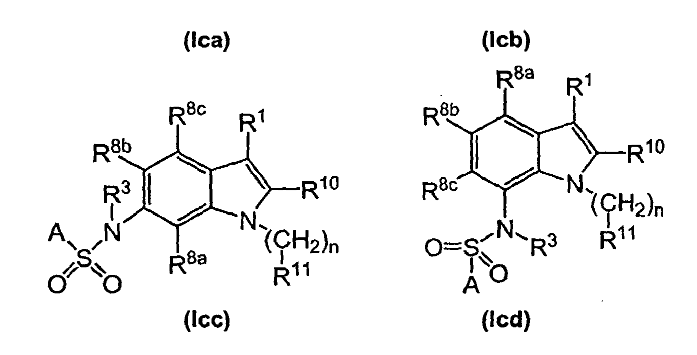 and wherein A, R¹, R³, R¹⁰, R¹¹, R¹², m and n have the meaning given above and wherein R^{8a}, R^{8b}, R^{8c} have the meaning given above.

In a very preferred embodiment of the invention the compound binding to the 5HT6-receptor being used according to the invention is selected from sulfonamide compounds according to formula Iba 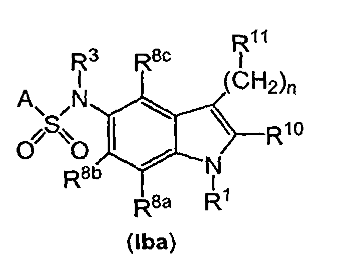 wherein
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;
- **R^{8a}, R^{8b}, R^{8c}**: each represent a hydrogen atom;
- **R¹⁰**: represents a hydrogen atom;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
- **n**: being 0, 1, 2, 3 or 4;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, =NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulphur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds according to formula (Iba) are known from WO03/042175 A1 and are well suitable to be selected to be used according to the invention, and thus the content of this publication referred to is in its entirety forming part of the description of this invention by reference.

It is further preferred if the compound binding to the 5HT6-receptor being used according to the invention is selected from the following group of sulfonamide compounds according to formula lba consisting of:
[1] N-[3-(2-diethylaminoethyl)-1H-indol-5-yl]-5-chloro-3-methylbenzo[b]thiophene-2-sulphonamide.
[2] N-[3-(2-diethylaminoethyl)-1H-indol-5-yl]naphthalene-1-sulphonamide.
[3] Hydrochloride N-[3-(2-diethylaminoethyl)-1*H*-indol-5-yl]naphthalene-1-sulphonamide.
[4] N-[3-(2-diethylaminoethyl)-1*H*-indol-5-yl]-3,5-dichlorobenzenesulphonamide.
[5] N-[3-(2-diethylaminoethyl)-1*H*-indol-5-yl]-4-phenylbenzenesulphonamide.
[6] N-[3-(2-diethylaminoethyl)-1*H*-indol-5-yl]-5-chlorothiophene-2-sulphonamide.
[7] N-[3-(2-dimethylaminoethyl)-1*H*-indol-5-yl]-5-chloro-3-methylbenzo[b]thiophene-2-sulphonamide.
[8] N-[3-(2-dimethylaminoethyl)-1*H*-indol-5-yl]naphthalene-1-sulphonamide.
[9] N-[3-(2-dimethylamino-ethyl)-1*H*-indol-5-yl]-6-chloroimidazo[2,1-b]thiazol-5-sulphonamide.
[10] N-[3-(1-methylpiperidin-4-yl)-1*H*-indol-5-yl]-5-chloro-3-methylbenzo[b]thiophene-2-sulphonamide.
[11] N-[3-(1-methylpiperidin-4-yl)-1*H*-indol-5-yl]-5-chloro-3-methylbenzo[b]thiophene-2-sulphonamide hydrochloride.
[12] N-[3-(1-methylpiperidin-4-yl)-1*H*-indol-5-yl]naphthalene-1-sulphonamide.
[13] N-[3-(1-methylpiperidin-4-yl)-1*H*-indol-5-yl]naphthalene-1-sulphonamide hydrochloride.
[14] N-[3-(1-methylpiperidin-4-yl)-1*H*-indol-5-yl]-5-chlorothiophene-2-sulphonamide.
[15] N-[3-(1-methylpiperidin-4-yl)-1*H*-indol-5-yl]-4-phenylbenzenesulphonamide.
[16] N-[3-(1-methylpiperidin-4-yl)-1*H*-indol-5-yl]quinoline-8-sulphonamide.
[17] N-[3-(2-diethylaminoethyl)-1*H*-indol-5-yl]naphthalene-2-sulphonamide.
[18] N-[3-(1-methyl-1,2,3,6-tetrahydropyridin-4-yl)-1*H*-indol-5-yl]naphthalene-1-sulphonamide.
[19] N-[3-(4-methylpiperazin-1-yl)methyl-1*H*-indol-5-yl]-5-chloro-3-methylbenzo[b]thiophene-2-sulphonamide.
[20] N-[3-(2-dimethylaminoethyl)-1*H*-indol-5-yl]-5-(2-pyridil)thiophene-2-sulphonamide.
[21] N-[3-(2-dimethylaminoethyl)-1*H*-indol-5-yl]-2,1,3-benzothiadiazol-4-sulphonamide.
[22] N-[3-(2-dimethylaminoethyl)-1*H*-indol-5-yl]quinoline-8-sulphonamide.
[23] N-[3-(2-dimethylaminoethyl)-1*H*-indol-5-yl]-5-chloronaphthalene-2-sulphonamide.
[24] N-[3-(2-dimethylaminoethyl)-1*H*-indol-5-yl]-4-phenoxybenzenesulphonamide.
[25] N-[3-(2-dimethylaminoethyl)-1*H*-indol-5-yl]-4-phenylbenzenesulphonamide.
[26] N-[3-(2-diethylaminoethyl)-1*H*-indol-5-yl]-N-ethyl-naphthalene-2-sulphonamide.
[27] N-{3-[2-(morpholin-4-yl)ethyl]-1H-indol-5-yl}-5-chloro-3-methylbenzo[b]thiophene-2-sulphonamide.
[28] N-{3-[2-(morpholin-4-yl)ethyl]-1H-indol-5-yl}naphthalene-1-sulphonamide.
[29] N-[3-(2-diethylaminoethyl)-1H-indol-5-yl]naphthalene-2-sulphonamide.
[30] N-[3-dimethylaminomethyl-1H-indol-5-yl]-5-chloro-3-methylbenzo[b]thiophene-2-sulphonamide.
[31] N-[3-(2-dipropylaminoethyl)-1H-indol-5-yl]naphthalene-1-sulphonamide.
[32] N-[3-(2-dipropylaminoethyl)-1H-indol-5-yl]-5-chloro-3-methylbenzo[b]thiophene-2-sulphonamide.
[33] N-[3-(2-dibutylaminoethyl)-1H-indol-5-yl]-5-chloro-3-methylbenzo[b]thiophene-2-sulphonamide.
[34] N-[3-(2-dibutylaminoethyl)-1H-indol-5-yl]naphthalene-1-sulphonamide.
[35] N-[3-(2-diethylaminoethyl)-1H-indol-5-yl]-5-chloronaphthalene-1-sulphonamide.
[36] N-[3-(2-diethylaminoethyl)-1H-indol-5-yl]-trans-β-styrenesulphonamide.
[37] N-[3-(4-methylpiperazin-1-yl)methyl-1*H*-indol-5-yl]-trans-β-styrenesulphonamide.
[38] N-[3-(octahydroindolizin-7-yl)-1H-indol-5-yl]-5-chloro-3-methylbenzo[b]thiophene-2-sulphonamide.
[39] N-[3-(2-diethylaminoethyl)-1*H*-indol-5-yl]-6-chloroimidazo[2,1-b]thiazol-5-sulphonamide.
[40] N-{3-[2-(morpholin-4-yl)ethyl]-1H-indol-5-yl}naphthalene-2-sulphonamide.
[41] N-[3-(4-methylpiperazin-1-yl)methyl-1*H*-indol-5-yl]-α-toluenesulphonamide.
[42] N-[3-(3-diethylaminopropyl)-1H-indol-5-yl]naphthalene-2-sulphonamide.
[43] N-[3-(3-diethylaminopropyl)-1H-indol-5-yl]-5-chloro-3-methylbenzo[b]thiophene-2-sulphonamide.
[44] N-{3-[2-(pyrrolidin-1-yl)ethyl]-1H-indol-5-yl}-5-chloro-3-methylbenzo[b]thiophene-2-sulphonamide.
[45] N-{3-[2-(pyrrolidin-1-yl)ethyl]-1H-indol-5-yl}naphthalene-1-sulphonamide.
[46] N-{3-[2-(pyrrolidin-1-yl)ethyl]-1H-indol-5-yl}naphthalene-2-sulphonamide.
[47] N-[3-(2-dipropylaminoethyl)-1H-indol-5-yl]naphthalene-2-sulphonamide.
[48] N-[3-(2-dimethylaminoethyl)-1H-indol-5-yl]-5-chloronaphthalene-1-sulphonamide.
[49] N-[3-(2-dimethylaminoethyl)-1H-indol-5-yl]naphthalene-2-sulphonamide.
[50] N-{3-[2-(morpholin-4-yl)ethyl]-1H-indol-5-yl}quinoline-8-sulphonamide.
[51] N-{3-[2-(morpholin-4-yl)ethyl]-1H-indol-5-yl}-4-phenylbenzenesulphonamide.
[52] N-[3-(4-methylpiperazin-1-yl)ethyl-1*H*-indol-5-yl]naphthalene-2-sulphonamide.
[53] N-[3-(4-methylpiperazin-1-yl)ethyl-1*H*-indol-5-yl]-5-chloronaphthalene-1-sulphonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the invention the compound binding to the 5HT6-receptor being used according to the invention is selected from sulfonamide compounds according to formula Ibb 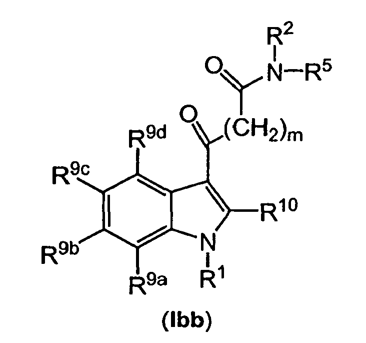 wherein
one of **R^{9a}, R^{9b}, R^{9c}, or R^{9d}** represents N(R³)-S(O₂)-A, or N-(S(O₂)-A)₂;
- A: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;
- **R^{9a}, R^{9b}, R^{9c}, R^{9d}** -: if not N(R³)-S(O₂)-A or N-(S(O₂)-A)₂- independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl; -O-C₁₋₅-alkyl; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH;
- **R¹⁰**: represents a hydrogen atom; a linear or branched optionally at least mono-substituted C₁₋₅-alkyl radical;
- m: represents 0, 1, 2, 3 or 4;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulphur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds according to formula (Ibb) are known from WO06/015867 A1 and are well suitable to be selected to be used according to the invention, and thus the content of this publication referred to is in its entirety forming part of the description of this invention by reference.

It is further preferred if the compound binding to the 5HT6-receptor being used according to the invention is selected from the following group of sulfonamide compounds according to formula Ibb consisting of:
2-[5-(5-Chloro-3-methyl-benzo[b]thiophene-2-sulfonylamino)-1H-indol-3-yl]-N,N-diethyl-2-oxoacetamide.
N,N-Diethyl-2-[5-(naphthalene-2-sulfonylamino)-1H-indol-3-yl]-2-oxo-acetamide.
N,N-Diethyl-2-[5-(naphtalene-1-sulfonylamino)-1H-indol-3-yl]-2-oxo-acetamide. 2-[5-(Biphenyl-4-sulfonylamino)-1H-indoi-3-yl]-N,N-diethyl-2-oxo-acetamide.
N,N-Diethyl-2-oxo-2-[5-(quinoline-8-sulfonylamino)-1H-indol-3-yl]-acetamide.
N,N-Dimethyl-2-[5-(naphthalene-2-sulfonylamino)-1H-indol-3-yl]-2-oxo-acetamide.
N,N-Dimethyl-2-[5-(naphtalene-1-sulfonylamino)-1H-indol-3-yl]-2-oxo-acetamide.
2-[5-(5-Chloro-3-methyl-benzo[b]thiophene-2-sulfonyl-amino)-1H-indol-3-yl]-N,N-dimethyl-2-oxo-acetamide.
2-[5-(6-Chloro-imidazo[2,1-b]thiazole-5-sulfonylamino)-1H-indol-3-yl]-N, N-diethyl-2-oxo-acetamide.
2-[5-(6-Chloro-imidazo[2,1-b]thiazole-5-sulfonylamino)-1H-indol-3-yl]-N, N-dimethyl-2-oxo-acetamide.
N,N-Dimethyl-2-[4-(naphthalene-1-sulfonylamino)-1H-indol-3-yl]-2-oxo-acetamide.
2-[4-(5-Chloro-3-methyl-benzo[b]thiophene-2-sulfonylamino)-1H-indol-3-yl]-N,N-dimethyl-2-oxo-acetamide.
2-[4-(6-Chloro-imidazo[2,1-b]thiazole-5-sulfonylamino)-1H-indol-3-yl]-N,N-dimethyl-2-oxo-acetamide.
N,N-Dimethyl-2-[5-[(4-fluoro-3-methyl-phenyl)-1-sulfonylamino]-1H-indol-3-yl]-2-oxo-acetamide.
5-(3-Dimethylaminooxalyl-1H-indol-5-ylsulfamoyl)-3-methyl-benzofuran-2-carboxylic acid ethyl ester.
2-[5-(Biphenyl-4-sulfonylamino)-1H-indol-3-yl]-N,N-dimethyl-2-oxo-acetamide.
N,N-Dimethyl-2-oxo-2-[5-(2-oxo-2,3-dihydro-benzoxazole-6-sulfonylamino)-1H-indol-3-yl]-acetamide.
N,N-Dimethyl-2-oxo-2-[5-(2-oxo-2,3-dihydrobenzo[d]thiazole-6-sulfonamido)-1H-indol-3-yl]acetamide.
2-[5-[(4-Cyclohexyl-phenyl)-1-sulfonylamino]-1H-indol-3-yl]-N,N-dimethyl-2-oxo-acetamide.
N,N-Dimethyl-2-[5-[(4-phenoxy-phenyl)-1-sulfonylamino]-1H-indol-3-yl]-2-oxo-acetamide.
2-(5-(5-chloro-3-methylbenzo[b]thiophene-2-sulfonamido)-2-methyl-1H-indol-3-yl)-N,N-dimethyl-2-oxoacetamide.
2-(5-(6-chloroimidazo[2,1-b]thiazole-5-sulfonamido)-2-methyl-1H-indol-3-yl)-N,N-dimethyl-2-oxoacetamide.
2-(6-(5-chloro-3-methylbenzo[b]thiophene-2-sulfonamido)-1H-indol-3-yl)-N,N-dimethyl-2-oxoacetamide.
N,N-dimethyl-2-(6-(naphthalene-3-sulfonamido)-1H-indol-3-yl)-2-oxoacetamide.
2-(6-(biphenyl-4-sulfonamido)-1H-indol-3-yl)-N,N-dimethyl-2-oxoacetamide.
N,N-dimethyl-2-(6-(naphthalene-1-sulfonamido)-1H-indol-3-yl)-2-oxoacetamide.
N,N-dimethyl-2-(6-(2-(naphthalen-1-yl)ethylsulfonamido)-1H-indol-3-yl)-2-oxoacetamide.
N,N-dimethyl-2-oxo-2-(6-(4-phenoxyphenylsulfonamido)-1H-indol-3-yl)acetamide.
2-(6-(3,4-dichlorothiophene-2-sulfonamido)-1H-indol-3-yl)-N,N-dimethyl-2-oxoacetamide.
2-(6-(3,5-dichlorophenylsulfonamido)-1H-indol-3-yl)-N,N-dimethyl-2-oxoacetamide.
2-(6-(1-chloronaphthalene-6-sulfonamido)-1H-indol-3-yl)-N,N-dimethyl-2-oxoacetamide.
2-(6-(6-chloroimidazo[2,1-b]thiazole-5-sulfonamido)-1H-indol-3-yl)-N,N-dimethyl-2-oxoacetamide.
N,N-diethyl-2-(2-methyl-5-(5-methyl-1-phenyl-1H-pyrazole-4-sulfonamido)-1H-indol-3-yl)-2-oxoacetamide.
N,N-diethyl-2-(2-methyl-5-(1,3,5-trimethyl-1H-pyrazole-4-sulfonamido)-1H-indol-3-yl)2-oxoacetamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the invention the compound binding to the 5HT6-receptor being used according to the invention is selected from sulfonamide compounds according to formula Ibc 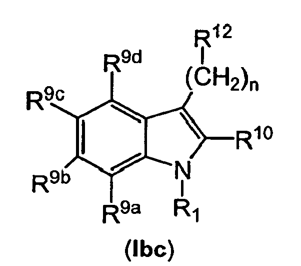 wherein
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents hydrogen;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ - if present - represents hydrogen;
- **R^{9a}, R^{9b}, R^{9c}, R^{9d}** -: if not N(R³)-S(O₂)-A or N-(S(O₂)-A)₂ - independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl; -O-C₁₋₅-alkyl; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; with the proviso that one of **R^{9a}, R^{9b}, R^{9c}, or R^{9d}** represents N(R³)-S(O₂)-A, or N-(S(O₂)-A)₂;
- **R¹⁰**: represents a hydrogen atom; a linear or branched optionally at least mono-substituted C₁₋₅-alkyl radical; preferably R¹⁰ represents hydrogen;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
- **R¹²**: represents R", or represents -C(OC₁₋₅-alkyl)-(CH₂)ₘ-R¹¹;
- **n**: being 0, 1, 2, 3 or 4;
- **m**: being 0, 1, 2, or 3;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃. - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds according to formula (Ibc) are known from WO06/024535 A1 and are well suitable to be selected to be used according to the invention, and thus the content of this publication referred to is in its entirety forming part of the description of this invention by reference.

It is further preferred if the compound binding to the 5HT6-receptor being used according to the invention is selected from the following group of sulfonamide compounds according to formula Ibc consisting of:
- 5-chloro-N-(3-(2-(diethylamino)ethyl)-1H-indol-6-yl)-3-methylbenzo[b]thiophene-2-sulfonamide
- N-(3-(2-(diethylamino)ethyl)-1H-indol-6-yl)naphthalene-2-sulfonamide
- N-(3-(2-(diethylamino)ethyl)-1H-indol-6-yl)naphthalene-1-sulfonamide
- 6-chloro-N-(3-(2-(diethylamino)ethyl)-1H-indol-6-yl)imidazo[2,1-b]thiazole-5-sulfonamide
- N-(3-(2-(diethylamino)ethyl)-1H-indol-6-yl)-4-phenylbenzenesulfonamide
- N-(3-(2-(diethylamino)ethyl)-1H-indol-6-yl)-4-phenoxybenzenesulfonamide
- 3,5-dichloro-N-(3-(2-(diethylamino)ethyl)-1H-indol-6-yl)benzenesulfonamide
- 4,5-dichloro-N-(3-(2-(diethylamino)ethyl)-1H-indol-6-yl)thiophene-2-sulfonamide
- 5-chloro-N-(3-(2-(diethylamino)ethyl)-1H-indol-6-yl)naphthalene-1-sulfonamide
- 5-chloro-N-(3-(2-(dimethylamino)ethyl)-1H-indol-6-yl)-3-methylbenzo[b]thiophene-2-sulfonamide
- N-(3-(2-(dimethylamino)ethyl)-1H-indol-6-yl)naphthalene-2-sulfonamide
- N -(3-(2-(dimethylamino)ethyl)-1H-indol-6-yl)naphthalene-1-sulfonamide
- 6-chloro-N-(3-(2-(dimethylamino)ethyl)-1H-indol-6-yl)imidazo[2,1-b]thiazole-5-sulfonamide
- N-(3-(2-(dimethylamino)ethyl)-1H-indol-6-yl)-4-phenylbenzenesulfonamide
- N-(3-(2-(dimethylamino)ethyl)-1H-indol-6-yl)-2-(naphthalen-1-yl)ethanesulfonamide
- N-(3-(2-(dimethylamino)ethyl)-1H-indol-6-yl)-4-phenoxybenzenesulfonamide
- 3,5-dichloro-N-(3-(2-(dimethylamino)ethyl)-1H-indol-6-yl)benzenesulfonamide
- 4,5-dichloro-N-(3-(2-(dimethylamino)ethyl)-1H-indol-6-yl)thiophene-2-sulfonamide
- 5-chloro-N-(3-(2-(dimethylamino)ethyl)-1H-indol-6-yl)naphthalene-1-sulfonamideo
- 5-chloro-N-(3-(2-(dimethylamino)-1-ethoxyethyl)-1H-indol-7-yl)-3-methylbenzo[b]thiophene-2-sulfonamide
- 5-chloro-N-(3-(2-(dimethylamino)ethyl)-1H-indol-7-yl)-3-methylbenzo[b]thiophene-2-sulfonamide
- 7-bis(5-chloro-3-methylbenzo[b]thiophene-2-sulfonyl)amino-3-(2-(diethylamino) -1-ethoxyethyl)-1 H-indole
- 5-chloro-N-(3-(2-(diethylamino)-1-ethoxyethyl)-1H-indol-7-yl)-3-methylbenzo[b]thiophene-2-sulfonamide
- 7-bis(5-chloro-3-methylbenzo[b]thiophene-2-sulfonyl)amino-3-(2-(dimethylamino)ethyl)-1H-indole
- 7-bis(5-chloro-3-methylbenzo[b]thiophene-2-sulfonyl)amino-3-(2-(diethylamino)ethyl)-1 H-indole
- 5-chloro-N-(3-(2-(diethylamino)ethyl)-1H-indol-7-yl)-3-methylbenzo[b]thiophene-2-sulfonamide
- 7-bis(6-chloroimidazo[2,1-b]thiazol-5-ylsulfonyl)amino-3-(2-(dimethylamino)ethyl)-1H-indole
- N-(3-(2-(dimethylamino)ethyl)-1H-indol-4-yl)-4-biphenylsulfonamide
- N-(3-(2-(dimethylamino)ethyl)-1H-indol-4-yl)-4-phenoxybenzenesulfonamide
- 3,5-dichloro-N-(3-(2-(dimethylamino)ethyl)-1H-indol-4-yl)benzenesulfonamide
- 5-chloro-N-(3-(2-(dimethylamino)ethyl)-1H-indol-4-yl)-3-methylbenzo[b]thiophene-2-sulfonamide
- N-(3-(2-(dimethylamino)ethyl)-1H-indol-4-yl)naphthalene-1-sulfonamide
- 5-chloro-N-(3-(2-(dimethylamino)ethyl)-1H-indol-4-yl)naphthalene-2-sulfonamide
- N-(3-(2-(dimethylamino)ethyl)-1H-indol-4-yl)naphthalene-2-sulfonamide
- 6-chloro-N-(3-(2-(dimethylamino)ethyl)-1H-indol-4-yl)imidazo[2,1-b]thiazole-5-sulfonamide
- N-(3-(2-(dimethylamino)ethyl)-1H-indol-4-yl)-2-(naphthalen-1-yl)ethanesulfonamide
- 6-bis(6-chloroimidazo[2,1-b]thiazol-5-ylsulfonyl)amino-3-(2-(dimethylamino)ethyl)-1H-indole
- 6-bis(3,5-dichlorobenzenesulfonyl)amino-3-(2-(dimethylamino)ethyl)-1H-indole
- 6-bis(4,5-dichlorothiophene-2-sulfonyl)amino-3-(2-(dimethylamino)ethyl)-1H-indole
- 6-bis(5-chloro-3-methylbenzo[b]thiophene-2-sulfonyl)amino-3-(2-(dimethylamino)-1-ethoxyethyl)-1H-indole
- N-(3-(2-(diethylamino)ethyl)-7-methoxy-1H-indol-5-yl)naphthalene-2-sulfonamide
- N-(3-(2-(diethylamino)ethyl)-7-methoxy-1H-indol-5-yl)benzo[c][1,2,5]thiadiazole-4-sulfonamide
- 6-chloro-N-(3-(2-(diethylamino)ethyl)-7-methoxy-1H-indol-5-yl)imidazo[2,1-b]thiazole-5-sulfonamide
- ethyl 6-(5-chloro-3-methylbenzo[b]thiophene-2-sulfonamido)-3-(1-methylpiperidin-4-yl)-1H-indole-5-carboxylate
- N-(5-bromo-3-(2-(pyrrolidin-1-yl)ethyl)-1H-indol-7-yl)-6-chloroimidazo[2,1-b]thiazole-5-sulfonamide
- N-(4-bromo-3-(1-methylpiperidin-4-yl)-1H-indol-6-yl)naphthalene-1-sulfonamide
- N-(7-bromo-3-(2-(dimethylamino)ethyl)-1H-indol-5-yl)benzofuran-2-sulfonamide
- N-(7-methoxy-3-(2-(pyrrolidin-1-yl)ethyl)-1H-indol-5-yl)benzo[c][1,2,5]thiadiazole-4-sulfonamide
- N-(7-methoxy-3-(2-(pyrrolidin-1-yl)ethyl)-1H-indol-5-yl)naphthalene-2-sulfonamide
- 6-chloro-N-(7-methoxy-3-(2-(pyrrolidin-1-yl)ethyl)-1H-indol-5-yl)imidazo[2,1-b]thiazole-5-sulfonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively;
preferably is selected from:
N-(3-(2-(diethylamino)ethyl)-7-methoxy-1H-indol-5-yl)naphthalene-2-sulfonamide,
N-(3-(2-(diethylamino)ethyl)-7-methoxy-1 H-indol-5-yl)benzo[c][1,2,5]thiadiazole-4-sulfonamide,
6-chloro-N-(3-(2-(diethylamino)ethyl)-7-methoxy-1H-indol-5-yl)imidazo[2,1-b]thiazole-5-sulfonamide,
ethyl 6-(5-chloro-3-methylbenzo[b]thiophene-2-sulfonamido)-3-(1-methylpiperidin-4-yl)-1H-indole-5-carboxylate,
N-(5-bromo-3-(2-(pyrrolidin-1-yl)ethyl)-1H-indol-7-yl)-6-chloroimidazo[2,1-b]thiazole-5-sulfonamide,
N-(4-bromo-3-(1-methylpiperidin-4-yl)-1H-indol-6-yl)naphthalene-1-sulfonamide,
N-(7-bromo-3-(2-(dimethylamino)ethyl)-1H-indol-5-yl)benzofuran-2-sulfonamide,
N-(7-methoxy-3-(2-(pyrrolidin-1-yl)ethyl)-1H-indol-5-yl)benzo[c][1,2,5]thiadiazole-4-sulfonamide,
N-(7-methoxy-3-(2-(pyrrolidin-1-yl)ethyl)-1H-indol-5-yl)naphthalene-2-sulfonamide and
6-chloro-N-(7-methoxy-3-(2-(pyrrolidin-1-yl)ethyl)-1H-indol-5-yl)imidazo[2,1-b]thiazole-5-sulfonamide.

In another preferred embodiment of the invention the compound binding to the 5HT6-receptor being used according to the invention is selected from sulfonamide compounds according to formula (Ica) 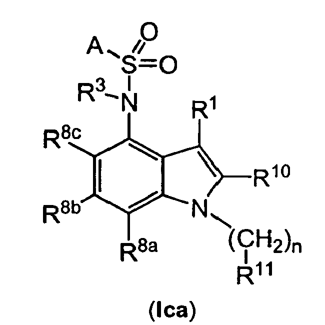 wherein
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
- **R^{8a}, R^{8b}, R^{8c}**: independently from one another, each represent a hydrogen atom; -NO₂;-NH₂; -SH; -OH; -CN; -C(=O)-H; -C(=O)-O-C₁₋₅-alkyl; -C(=O)-C₁₋₅-alkyl; -O-C₁₋₅-alkyl';-S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{8a}, R^{8b}, R^{8c}** each represent a hydrogen atom;
- **R¹⁰**: represents a hydrogen atom; a linear or branched optionally at least mono-substituted C₁₋₅-alkyl radical; preferably R¹⁰ represents a hydrogen atom;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
- **n**: being 0, 1, 2, 3 or 4; preferably n being 2;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds according to formula (Ica) are known from WO05/013978 A1 and are well suitable to be selected to be used according to the invention, and thus the content of this publication referred to is in its entirety forming part of the description of this invention by reference.

It is further preferred if the compound binding to the 5HT6-receptor being used according to the invention is selected from the following group of sulfonamide compounds according to formula (Ica) consisting of:
[1] N-[1-(2-dimethylaminoethyl)-1H-indole-4-yl]-5-chloro-3-methylbenzo[b]thiophene-2-sulfonamide,
[2] N-[1-(2-dimethylaminoethyl)-1H-indole-4-yl]-naphtalene-2-sulfonamide,
[3] N-[1-(2-dimethylaminoethyl)-1H-indole-4-yl]-naphtalene-1-sulfonamide,
[4] N-[1-(2-dimethylaminoethyl)-1H-indole-4-yl]-4-phenylbenzenesulfonamide,
[5] N-[1-(2-dimethylaminoethyl)-1H-indole-4-yl]-2-(naphtalene-1-yl)-ethanesulfonamide,
[6] N-[1-(2-dimethylaminoethyl)-1H-indole-4-yl]-4-phenoxybenzenesulfonamide,
[7] N-[1-(2-dimethylaminoethyl)-1H-indole-4-yl]-3,5-dichlorobenzenesulfonamide and
[8] 6-chloro-N-[1-(2-dimethylaminoethyl)-1H-indol-4-yl]-imidazo[2,1-b]thiazole-5-sulfonamide
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the invention the compound binding to the 5HT6-receptor being used according to the invention is selected from sulfonamide compounds according to formula (Icb) 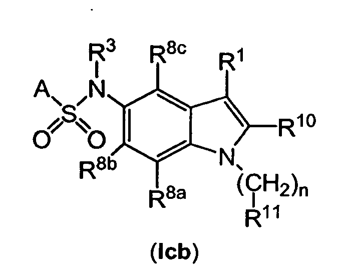 wherein
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
- **R^{8a}, R^{8b}, R^{8c}**: independently from one another, each represent a hydrogen atom; -NO₂;-NH₂; -SH; -OH; -CN; -C(=O)-H; -C(=O)-O-C₁₋₅-alkyl; -C(=O)-C₁₋₅-alkyl; -O-C₁₋₅-alkyr';-S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{8a}, R^{8b}, R^{8c}** each represent a hydrogen atom;
- **R¹⁰**: represents a hydrogen atom; a linear or branched optionally at least mono-substituted C₁₋₅-alkyl radical; preferably R¹⁰ represents a hydrogen atom or methyl;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
- **n**: being 0, 1, 2, 3 or 4; preferably n being 2;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds according to formula (Icb) are known from WO05/013977 A1 and are well suitable to be selected to be used according to the invention, and thus the content of this publication referred to is in its entirety forming part of the description of this invention by reference. It is further preferred if the compound binding to the 5HT6-receptor being used according to the invention is selected from the following group of sulfonamide compounds according to formula (Icb) consisting of:
[1] N-[1-(2-dimethylaminoethyl)-1H-indole-5-yl]-5-chloro-3-methylbenzo[b]thiophene-2-sulfonamide,
[2] N-[1-(2-dimethylaminoethyl)-1H-indole-5-yl]-naphthalene-2-sulfonamide,
[3] N-[1-(2-dimethylaminoethyl)-1H-indole-5-yl]-naphthalene-1-sulfonamide,
[4] N-[1-(2-dimethylaminoethyl)-1H-indole-5-yl]-5-chloronaphthalene-1-sulfonamide,
[5] N-[1-(2-dimethylaminoethyl)-1H-indole-5-yl]-benzenesulfonamide,
[6] N-[1-(2-dimethylaminoethyl)-1H-indole-5-yl]-quinoline-8- sulfonamide,
[7] N-[1-(2-dimethylaminoethyl)-1H-indole-5-yl]-4-phenoxybenzenesulfonamide,
[8] N-[1-(2-dimethylaminoethyl)-1H-indole-5-yl]-4-methylbenzenesulfonamide,
[9] N-[1-(2-dimethylaminoethyl)-1H-indole-5-yl]-5-chlorothiophene-2-sulfonamide,
[10] N-[1-(2-dimethylaminoethyl)-1H-indole-5-yl]-benzo[1,2,5]thiadiazole-4-sulfonamide,
[11] N-[1-(2-dimethylaminoethyl)-1H-indole-5-yl]-6-chloroimidazo[2,1-b]thiazole-5-sulfonamide,
[12] N-[1-(2-dimethylaminoethyl)-1H-indole-5-yl]-3,5-dichlorobenzenesulfonamide,
[13] N-[1-(2-dimethylaminoethyl)-1H-indole-5-yl]-3-bromobenzenesulfonamide,
[14] N-[1-(2-dimethylaminoethyl)-1H-indole-5-yl]-3-nitrobenzenesulfonamide,
[15] N-[1-(2-dimethylaminoethyl)-1H-indole-5-yl]-1-phenylmethanesulfonamide,
[16] N-[1-(2-pyrrolidine-1-yl-ethyl)-1H-indole-5-yl]-naphthalene-2-sulfonamide,
[17] N-[1-(2-pyrrolidine-1-yl-ethyl)-1H-indole-5-yl]-naphthalene-1-sulfonamide,
[18] N-[1-(2-pyrrolidine-1-yl-ethyl)-1H-indole-5-yl]-5-chloro-3-methylbenzo[b]thiophene-2-sulfonamide,
[19] trans-N-[1-(2-dimethylaminoethyl)-1H-indole-5-yl]-2-phenylethenesulfonamide,
[20] N-[1-(2-dimethylaminoethyl)-1H-indole-5-yl]-4,5-dichlorothiophene-2-sulfonamide,
[21] N-[1-(2-dimethytaminoethyl)-1H-indole-5-yl]-4-acetylbenzenesulfonamide,
[22] N-[1-(2-dimethylaminoethyl)-1H-indole-5-yl]-4-bromobenzenesulfonamide,
[23] N-[1-(2-dimethylaminoethyl)-1H-indole-5-yl]-4-methoxybenzenesulfonamide,
[24] N-[3-(2-diethylaminoethyl)-1H-indole-5-yl]-5-chloro-3-methylbenzo[b]thiophene-2-sulfonamide,
[25] N-[1-(2-dimethylaminoethyl)-1H-indole-5-yl]-4-nitrobenzenesulfonamide,
[26] N-[1-(2-dimethylaminoethyl)-1H-indole-5-yl]-4-fluorobenzenesulfonamide,
[27] N-[1-(2-diethylaminoethyl)-1H-indole-5-yl]-6-chloroimidazo[2,1-b]thiazole-5-sulfonamide,
[28] N-[1-(2-pyrrolidine-1-yl-ethyl)-1H-indole-5-yl]- ]-6-chloroimidazo[2,1-b]thiazole-5-sulfonamide,
[29] N-(1-(2-(diethylamino)ethyl)-1H-indol-5-yl)-naphthalene-2-sulfonamide,
[30] N-(1-(2-(diethylamino)ethyl)-1H-indol-5-yl)-naphthalene-1-sulfonamide,
[31] N-(1-(2-(diethylamino)ethyl)-1H-indol-5-yl)-4-phenylbenzenesulfonamide,
[32] 5-chloro-N-(1-(2-(dimethylamino)ethyl)-2-methyl-1H-indol-5-yl)-3-methylbenzo[b]thiophene-2-sulfonamide,
[33] N-(1-(2-(dimethylamino)ethyl)-2-methyl-1H-indol-5-yl)-naphthalene-2-sulfonamide,
[34] N-(1-(2-(dimethylamino)ethyl)-2-methyl-1H-indol-5-yl)-naphthalene-1-sulfonamide,
[35] 6-chloro-N-(1-(2-(dimethylamino)ethyl)-2-methyl-1H-indol-5-yl)imidazo[2,1-b]thiazole-5-sulfonamide,
[36] N-(1-(2-(dimethylamino)ethyl)-2-methyl-1H-indol-5-yl)-4-phenylbenzenesulfonamide,
[37] N-(1-(2-dimethylamino)ethyl)-2-methyl-1H-indol-5-yl)-2-(naphth-1-yl)-ethanesulfonamide,
[38] N-(1-(2-(dimethylamino)ethyl)-2-methyl-1H-indol-5-yl)-4-phenoxy-benzenesulfonamide,
[39] 3,5-dichloro-N-(1-(2-(dimethylamino)ethyl)-2-methyl-1H-indol-5-yl)-benzenesulfonamide,
[40] N-(1-(2-(dimethylamino)ethyl)-2-methyl-1H-indol-5-yl)benzo[b]thiophene-3-sulfonamide,
[41] N-(1-(2-(diethylamino)ethyl)-1H-indol-5-yl)benzo[b]thiophene-3-sulfonamide and
[42] N-(1-(2-(dimethylamino)ethyl)-1H-indol-5-yl)benzo[b]thiophene-3-sulfonamide,
[43] 5-chloro-3-methyl-N-(1-(3-(piperidin-1-yl)propyl)-1H-indol-5-yl)benzo[b]thiophene-2-sulfonamide,
[44] N-(1-(3-(piperidin-1-yl)propyl)-1H-indol-5-yl)naphthalene-2-sulfonamide,
[45] N-(1-(3-(piperidin-1-yl)propyl)-1H-indol-5-yl)naphthalene-1-sulfonamide,
[46] 6-chloro-N-(1-(3-piperidin-1-yl)propyl)-1H-indol-5-yl)imidazo[2,1-b]thiazole-5-sulfonamide,
[47] 4-phenyl-N-(1-(3-(piperidin-1-yl)propyl)-1H-indol-5-yl)benzenesulfonamide,
[48] 2-(naphth-1-yl)-N-(1-(3-(piperidin-1-yl)propyl)-1H-indol-5-yl)ethanesulfonamide,
[49] 4-phenoxy-N-(1-(3-(piperidin-1-yl)propyl)-1H-indol-5-yl)benzenesulfonamide,
[50] 3,5-dichloro-N-(1-(3-(piperidin-1-yl)propyl)-1H-indol-5-yl)benzenesulfonylamide,
[51] 4,5-dichloro-N-(1-(3-(piperidin-1-yl)propyl)-1H-indol-5-yl)thiophene-2-sulfonamide and
[52] 5-chloro-N-(1-(3-(piperidin-1-yl)propyl)-1H-indol-5-yl)naphthalene-1-sulfonamide,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the invention the compound binding to the 5HT6-receptor being used according to the invention is selected from sulfonamide compounds according to formula (Icc) 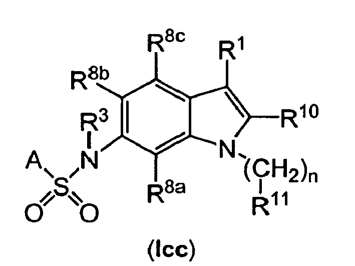 wherein
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
- **R^{8a},**: **R^{8b}, R^{8c}** independently from one another, each represent a hydrogen atom; -NO₂;-NH₂; -SH; -OH; -CN; -C(=O)-H; -C(=O)-O-C₁₋₅-alkyl; -C(=O)-C₁₋₅-alkyl; -O-C₁₋₅-alkyl';-S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{8a}, R^{8b}, R^{8c}** each represent a hydrogen atom;
- **R¹⁰**: represents a hydrogen atom; a linear or branched optionally at least mono-substituted C₁₋₅-alkyl radical; preferably R¹⁰ represents a hydrogen atom;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
- n: being 0, 1, 2, 3 or 4; preferably n being 2;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulphur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds according to formula (Icc) are known from WO05/013976 A1 and are well suitable to be selected to be used according to the invention, and thus the content of this publication referred to is in its entirety forming part of the description of this invention by reference. It is further preferred if the compound binding to the 5HT6-receptor being used according to the invention is selected from the following group of sulfonamide compounds according to formula (Icc) consisting of:
[1] N-[1-(2-Dimethylaminoethyl)-1H-indol-6-yl]-5-chloro-3-methylbenzo[b]thiophene-2-sulfonamide,
[2] N-[1-(2-Dimethylaminoethyl)-1H-indol-6-yl]-naphthalene-2-sulfonamide,
[3] N-[1-(2-Dimethylaminoethyl)-1H-indol-6-yl]-naphthalene-1-sulfonamide,
[4] N-[1-(2-Dimethylaminoethyl)-1H-indol-6-yl]-6-chloroimidazo[2,1-b]thiazole-5-sulfonamide,
[5] N-[1-(2-Dimethylaminoethyl)-1H-indol-6-yl]-4-phenylbenzenesulfonamide,
[6] N-[1-(2-Dimethylaminoethyl)-1H-indol-6-yl]-2-(naphthalene-1-yl)-ethanesulfonamide,
[7] N-[1-(2-Dimethylaminoethyl)-1H-indol-6-yl]-4-phenoxybenzenesulfonamide,
[8] N-[1-(2-Dimethylaminoethyl)-1H-indol-6-yl]-3.5-dichlorobenzenesulfonamide,
[9] 5-Chloro-3-methyl-N-[1-[2-(pyrrolidin-1-yl)ethyl-1H-indol-6-yl]-benzo[b]thiophene-2-sulfonamide,
[10] N-(1-[2-(Pyrrolidin-1-yl)ethyl]-1H-indol-6-yl]-napthalene-2-sulfonamide,
[11] N-[1-[2-Pyrrolidin-1-yl]ethyl]-1H-indol-6-yl]-naphthalene-1-sulfonamide,
[12] 6-Chloro-N-[1-[2-(pyrrolidin-1-yl)ethyl]-1H-indol-6-yl]-imidazo[2,1-b]thiazole-5-sulfonamide,
[13] 4-Phenyl-N-(1-(2-(pyrrolidin-1-yl)ethyl)-1H-indol-6-yl)-benzenesulfonamide
[14] 2-(Naphthyl-1-yl)-N-(1-(2-(pyrrolidin-1-yl)ethyl)-1H-indol-6-yl)-ethansulfonamide,
[15] 4-Phenoxy-N-(1-(2-(pyrrolidin-1-yl)ethyl)-1H-indol-6-yl)-benzenesulfonamide and
[16] 3,5-Dichloro-N-(1-(2-(pyrrolidin-1-yl)-1H-indol-6-yl)-benzenesulfonamide,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the invention the compound binding to the 5HT6-receptor being used according to the invention is selected from sulfonamide compounds according to formula (Icd) 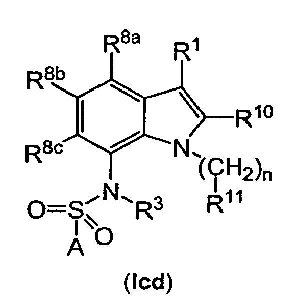 wherein
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NLO₂, -CN, -NH-CH₃ and -S-CH₃; or benzyl; preferably R¹ represents a hydrogen atom;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
- **R^{8a}**, **R^{8b}, R^{8c}**: independently from one another, each represent a hydrogen atom; -NO₂;-NH₂; -SH; -OH; -CN; -C(=O)-H; -C(=O)-O-C₁₋₅-alkyl; -C(=O)-C₁₋₅-alkyl; -O-C₁₋₅-alkyl;-S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{8a}, R^{8b}, R^{8c}** each represent a hydrogen atom;
- **R¹⁰**: represents a hydrogen atom; a linear or branched optionally at least mono-substituted C₁₋₅-alkyl radical; preferably R¹⁰ represents a hydrogen atom;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
- **n**: being 0, 1, 2, 3 or 4; preferably n being 2;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅; or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I. -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulphur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds according to formula (Icd) are known from WO05/013979 A1 and are well suitable to be selected to be used according to the invention, and thus the content of this publication referred to is in its entirety forming part of the description of this invention by reference.

It is further preferred if the compound binding to the 5HT6-receptor being used according to the invention is selected from the following group of sulfonamide compounds according to formula (Icd) consisting of:
[1] N-[1-(2-dimethylaminoethyl)-1H-indole-7-yl]-naphtalene-1-sulfonamide,
[2] N-[1-(2-dimethylaminoethyl)-1H-indole-7-yl]-5-chloro-3-methylbenzo[b]thiophene-2-sulfonamide,
[3] N-[1-(2-dimethylaminoethyl)-1H-indole-7-yl]-4-phenylbenzenesulfonamide and
[4] N-[1-(2-dimethylaminoethyl)-1H-indole-7-yl]-6-chloroimidazo[2,1-b]thiazole-5-sulfonamide
[5] 5-chloro-3-methyl-N-(1-(2-(pyrrolidin-1-yl)ethyl)-1H-indol-7-yl)-benzo[b]thiophen-2-sulfonamide,
[6] N-(1-(2-(pyrrolidin-1-yl)ethyl)-1H-indol-7-yl)naphthalene-1-sulfonamide,
[7] 6-chloro-N-(1-(2-(pyrroldin-1-yl)ethyl)-1H-indol-7-yl)imidazo[2,1-b]thiazole-5-sulfonamide and
[8] 2-(naphth-1-yl)-N-(1-(2-(pyrrolidin-1-yl)ethyl)-1H-indol-7-yl)ethansulfonamide
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the invention the compound binding to the 5HT6-receptor being used according to the invention is selected from sulfonamide compounds according to formula (Id) 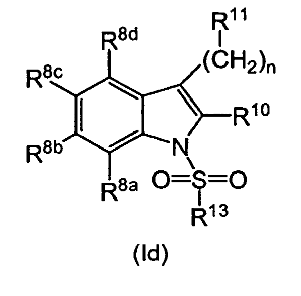 wherein
- **R^{8a}, R^{8b}, R^{8c}**, **R^{8d}**: independently from one another, each represent a hydrogen atom;-NO₂; -NH₂; -SH; -OH; -CN; -C(=O)-H; -C(=O)-O-C₁₋₅-alkyl; -C(=O)-C₁₋₅-alkyl; -O-C₁₋₅-alkyl; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH;
- **R¹⁰**: represents a hydrogen atom;; a linear or branched optionally at least mono- substituted C₁₋₅-alkyl radical; preferably R2 represents H;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
- n: being 0, 1, 2, 3 or 4;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅; or
- **R²** and **R⁵**: together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃,-OCF₃, - -OH, -SH, -NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃, -SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively

Compounds according to formula (Id) are known from WO05/013974 A1 and are well suitable to be selected to be used according to the invention, and thus the content of this publication referred to is in its entirety forming part of the description of this invention by reference.

It is further preferred if the compound binding to the 5HT6-receptor being used according to the invention is selected from the following group of sulfonamide compounds according to formula (Id) consisting of:
[1] 1-Cyclohexanesulfonyl-3-(1-methyl-1,2,3,6-tetrahydropyridine-4-yl)-5-nitro-1H-indole,
[2] 5-Chloro-1-cyclohexanesulfonyl-3-(1-methyl-1,2,3,6-tetrahydropyridine-4-yl)-1H-indole,
[3] 5-Amino-1-cyclohexanesulfonyl-3-(1-methyl-1,2,3,6-tetrahydropyridine-4-yl)-1H-indole and
[4] 1-Cyclohexanesulfonyl-5-fluoro-3-(1,2,3,5,8,8a-hexahydro-indolizine-7-yl)-1H-indole hydrochloride;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the invention the compound binding to the 5HT6-receptor being used according to the invention is selected from sulfonamide compounds according to formula (Ie) 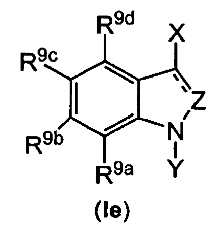 wherein
**wherein**
**--Z** either represents -CH₂ or =N;
   **and wherein**
**X** represents R¹, while **Y** represents (CH₂)ₙ-R¹¹ or
**Y** represents R¹, while **X** represents (CH₂)ₙ-R¹¹;
   while
one of R^{9a}, R^{9b}, R^{9c}, or R^{9d} represents N(R³)-S(O₂)-A;
**and wherein**
- **A**: represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
- **R¹**: represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
- **R³**: represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably **R³** represents a hydrogen atom;
- **R^{9a}, R^{9b}, R^{9c}, R^{9d}** -: if not N(R³)-S(O₂)-A - independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl; -O-C₁₋₅-alkyl; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{9a}**, **R^{9b}, R^{9c}, R^{9d}** - if not N(R³)-S(O₂)-A - each represent a hydrogen atom;
- **R¹¹**: represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
- **n**: being 0, 1, 2, 3 or 4;
- **R²**: represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
- **R⁵**: represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅; or
- **R²**: and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Compounds according to formula (Ie) are known from WO06/069809 A1 and are well suitable to be selected to be used according to the invention, and thus the content of this publication referred to is in its entirety forming part of the description of this invention by reference.

It is further preferred if the compound binding to the 5HT6-receptor being used according to the invention is selected from the following group of sulfonamide compounds according to formula (Ie) consisting of:
[1] N-(1-(2-(Dimethylamino)ethyl)-1H-indazol-6-yl)napthalene-2-sulphonamide;
[2] 5-Chloro-N-(1-(2-(dimethylamino)ethyl)-1H-indazol-6-yl)-3-methylbenzo[b]thiophene-2-sulfonamide;
[3] Naphthalene-2-sulfonic acid [3-(1-methyl-piperidin-4-yl)-1H-indazol-5-yl]-amide,
[4] 5-Chloro-3-methyl-benzo[b]thiophene-2-sulfonic acid [3-(1-methyl-piperidin-4-yl)-1H-indazol-5-yl]-amide,
[5] Naphthalene-1-sulfonic acid [3-(1-methyl-piperidin-4-yl)-1H-indazol-5-yl]-amide,
[6] 4-Phenylbenzene-4-sulfonic acid [3-(1-methyl-piperidin-4-yl)-1H-indazol-5-yl]-amide,
[7] N-[3-(1-Methyl-piperidin-4-yl)-1H-indazol-5-yl]-4-phenoxy-benzenesulfonamide
[8] N-[3-(1-Methyl-piperidin-4-yl)-1H-indazol-5-yl]-benzenesulfonamide;
[9] N-[1-(2-Dimethylamino)ethyl)-2,3-dihydro-1H-indol-6-yl]-6-chloro-imidazo[2,1-b]thiazol-5-sulfonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In a preferred embodiment of the use according to the invention the medicament is for the treatment of weight-gain induced by a therapeutic drug.

In another preferred embodiment of the use according to the invention the medicament is for the treatment of obesity induced by a therapeutic drug.

In another preferred embodiment of the use according to the invention the medicament is for the treatment of cardio-metabolic risk factors induced by a therapeutic drug.

A typical example of a therapeutic drug causing/inducing weight gain is olanzapine. This is a drug that is known to cause weight-gain, obesity and associated cardio-metabolic risk.
Examples of other antipsychotic drugs that cause weight-gain are the atypical antipsychotics including, but not limited to, olanzapine clozapine, quetiepine, risperidone, amisulpiride, zotepine, sertindole, aripiprazole and ziprasidone, and also the older typical neuroleptics including, but not limited to, chlorpromazine, haloperidol, fluphenazine, mesoridazine and thioridazine.

Typical and atypical antipsychotics are not the only classes of drugs that have the potential to cause marked weight-gain and obesity. It is well known by those skilled in the art that many other drugs cause weight-gain and obesity, and as a consequence, put patients receiving these medicaments at increased cardio-metabolic risk (eg see review by Pijl & Meinders, 1996).

Many other classes of drugs other than typical and atypical antipsychotics cause weight gain in patients. Examples of therapeutic classes and specific examples of (therapeutic) drugs causing weight-gain include, but are not limited to, the following:
Antidepressants, comprising the non-selective, irreversible monoamine oxidase A and B inhibitors (MAOls) including, but not limited to, tranylcypromine, phenelzine and isocarboxacid, the tricyclic antidepressants (TCAs) including, but not limited to, amitriptyline, amoxapine, clomipramine, desipramine, doxepin, imiprimine, nortriptyline, protriptyline and trimiprimine, the serotonin-selective reuptake inhibitors including, but not limited to, fluoxetine, sertraline, paroxetine and citalopram, the so-called "third generation" and atypical antidepressants including, but not limited to, mirtazepine, venlafaxine, mianserin and trazodone.
Drugs to treat mania in bipolar disorder including, but not limited to, lithium, olanzapine, quetiepine and sodium valproate.
Corticosteroids to treat inflammation including, but not limited to, prednisone, prednisolone, budesonide, dexamethasone, methylprednisolone and betamethasone Drugs for the treatment of Type 1 diabetes, insulin resistance, impaired glucose tolerance and Type 2 diabetes including, but not limited to the sulphonylureas (chlorpropamide, tolbutamide, acetoheximide, tolazamide, glibenclamide, glipizide, gliclazide, glimepiride, gliquidone, glyburide), the meglitinides (netaglinide, repaglinide, mitiglitinide), peroxisome proliferator activated receptors type gamma (PPARγ) agonists (troglitazone, rosiglitazone, pioglitazone, ciglitazone, darglitazone), inhibitors of hepatic glucose production (glucagon antagonists, glucose-6-phosphatase inhibitors, glycogen phosphorylase inhibitors), insulin (human insulin lispro, human insulin aspart, neutral insulin, human neutral insulin [pyr], human neutral insulin [prb], bovine neutral insulin, porcine neutral insulin, human isophane insulin, isophase insulin [prb], human isophane insulin [pyr], porcine isophane insulin, bovine isophane insulin, human insulin zinc suspension [pyr], humulin [prb], porcine insulin zinc suspension, human insulin zinc suspension [prb], human insulin glargine, protamine zinc insulin injection [prb], bovine insulin zinc suspension, bovine protamine zinc insulin, biphasic human insulin [pyr], biphasic human insulin [prb], biphasic neutral and isophane human insulin [prb], human insulin [emp], biphasic porcine neutral and isophane insulin, biphasic insulin aspart, insulin determir), dual-acting insulin secretagogues/insulin sensitisers (pituitary adenylate cyclase-activating peptide/ vasoactive intestinal peptide receptor [VPAC] 2 receptor agonists), modulators of glucose uptake (GLUT 4 translocators, retinoid X receptor agonists, Iκ β kinase [IKK] β inhibitors, p38 MAP kinase inhibitors, protein tyrosine phosphatase 1 B [PTP1 B] inhibitors, insulin receptor tyrosine kinase activators, α-glucosidase inhibitors, sodium/glucose co-transport inhibitors, glycogen synthase kinase 3 [GSK3] inhibitors) and modulators of mitochondrial metabolism. Medications may also comprise combinations of the above listed compounds, including, but not limited to rosiglitazone + metformin, rosiglitazone + simvastatin, rosiglitazone + sulphonurea, metformin + glipizide, sitagliptin + metformin, PPARγ/α combination medications (sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar, netoglitazone), PPARγ/α/δ combination medications.
Antihypertensives including, but not limited to, β-adrenoceptor blockers, eg propranolol, metoprolol and atenolol, and α₁-adrenoceptor blockers, eg doxasin and prazosin.
Drugs for the treatment of gastro-oesophogeal reflux, dyspepsia and ulcers including, but not limited to, esomeprazole, omeprazole and lansoprazole.
Drugs for the treatment and prevention of epilepsy and seizure disorders including, but not limited to, vigabatrin, piracetam, carbamazepine, pregabalin, gabapentin, lamotrigine, and sodium valproate.
Drugs for the treatment or prevention of neuropathic pain including, but not limited to, carbamazepine, pregabalin, gabapentin, sodium valproate, lamotrigine and the tricyclic antidepressants, eg amitriptyline, amoxapine, clomipramine, desipramine, doxepin, imiprimine, nortriptyline, protriptyline and trimiprimine, or atypical and "third generation" antidepressant drugs, eg mirtazepine, venlafaxine, mianserin and trazodone.
Drugs for the treatment and prevention of migraine including, but not limited to, methysergide,
   pizotifen, memantine, antiepileptic drugs, eg vigabatrin, piracetam, carbamazepine, pregabalin, gabapentin, lamotrigine, and sodium valproate, tricyclic antidepressants, eg amitriptyline, amoxapine, clomipramine, desipramine, doxepin, imiprimine, nortriptyline, protriptyline and trimiprimine, the serotonin-selective reuptake inhibitors including, but not limited to, fluoxetine, sertraline, paroxetine and citalopram, and atypical and "third generation" antidepressant drugs, eg mirtazepine, venlafaxine, mianserin and trazodone.
Antihistamines including, but not limited to, diphenhydramine and chlorpheniramine.
Benzodiazepines including, but not limited to, clobazam.
Contraceptive medication, particularly injectable depot contraceptives including, but not limited to, depot medroxyprogesterone acetate (DMPA)
Hormone replacement therapy including, but not limited to, synthetic or natural oestrogen and progesterone and their analogues administered either alone or in combination.
Thus, In a preferred embodiment of the use according to the invention the therapeutic drug inducing weight gain, obesity or associated cardio-metabolic risk factors is selected rom the group consisting of:
   antipsychotics, comprising the atypical antipsychotics and typical neuroleptics, antidepressants including MAOls, TCAs, SSRls, "third generation" and
   atypical antidepressants, anti-manic agents, corticosteroids, agents for insulin resistance, impaired glucose tolerance and Type 2 diabetes, antihypertensives, anti-gastric ulceratives, anti-epileptics, drugs used in
      medicaments for neuropathic pain, antihistamines, drugs used in medicaments for migraine, benzodiazepines, contraceptives and drugs used in hormone replacement therapy.

In a preferred embodiment of the use according to the invention the therapeutic drug inducing weight gain, obesity or associated cardio-metabolic risk factors is an antipsychotic, comprising the atypical antipsychotics and typical neuroleptics, antidepressants including MAOls, TCAs, SSRls, "third generation" and atypical antidepressants, anti-manic agents. Especially the therapeutic drug inducing weight gain, obesity or associated cardio-metabolic risk factors is a neuroleptic.

The medicament may be in any form suitable for the application to humans and/or animals, preferably mammals, and can be produced by standard procedures known to those skilled in the art. The composition of the medicament may vary depending on the route of administration.

The medicament of the present invention may e.g. be administered parentally in combination with conventional injectable liquid carriers, such as water or suitable alcohols. Conventional pharmaceutical adjuvants for injection, such as stabilizing agents, solubilizing agents, and buffers, may be included in such injectable compositions. These medicaments may preferably be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered form suitable for reconstitution with water or other suitable liquid medium before use, for immediate or controlled release.

The liquid oral forms for administration may also contain certain additives such as sweeteners, flavoring, preservatives, and emulsifying agents. Non-aqueous liquid compositions for oral administration may also be formulated, containing e.g. edible oils. Such liquid compositions may be conveniently encapsulated in e.g., gelatin capsules in a unit dosage amount.

The compositions of the present invention may also be administered topically or via a suppository.

The above mentioned compositions include preferably 1 to 60 % by weight of one or more of the compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and 40 to 99 % by weight of the appropriate pharmaceutical vehicle(s).

The daily dosage of the 5HT6 ligand used according to the invention in the manifacure of a medicament for humans and animals may vary depending on factors that have their basis in the respective species or other factors, such as age, weight or degree of illness and so forth.
The daily dosage for mammals including humans usally ranges from 1 milligram to 2000 milligram, preferably 1 to 1500 mg, more preferably 1 to 1000 mg of substance to be administered during one or several intakes.

The medicament manufactured according to the invention may be applied in different modes, sometimes for the direct treatment of a symptom, sometimes as a prophylaxis against development of this symptom. In one embodiment the medicament being manufactured according to the invention is applied after the effects of the drug causing weight-gain, obesity and increase in associated cardio-metabolic risk factors has caused or induced an increase of body weight or associated cardio-metabolic risk factors in the patient. In another embodiment the medicament being manufactured according to the invention is applied after the drug causing weight gain, obesity and increase in associated cardio-metabolic risk factors has been applied, but before or during the onset of the increase in body-weight or associated cardio-metabolic risk factors. In a further embodiment the medicament being manufactured according to the invention is applied at the same time ( or approximately the same time) when the drug causing weight gain, obesity and increase in associated cardio-metabolic risk factors is applied. Then the application of the drug and the medicament manufactured according to the invention may be done through different pharmceutical pathways, e.g. orally/parentaral, or through the same pharmaceutical pathway. The application of the drug and the medicament manufactured according to the invention may then be done at the same or different sites of application or using distinct or the same pharmaceutical formulation or medicament. Thus, the application of the drug causing weight gain, obesity and increase in associated cardio-metabolic risk factors and of the 5HT6-Ligand used according to the invention in the the manufacture of the medicament may also be done inside the same pharmaceutical formulation. In a further embodiment the medicament being manufactured according to the invention is applied before the drug causing weight-gain, obesity and increase in associated cardio-metabolic risk factors is applied.

As a further aspect the invention also provides a method of treatment for weight gain, obesity and increase in associated cardio-metabolic risk factors caused or induced by a drug by applying to a mammal or patient in need thereof a suitable amount of a 5HT6 ligand in a medicament manufactured according to the invention. In one embodiment the medicament manufactured according to the invention may be applied in different modes. In one embodiment the medicament being manufactured according to the invention is applied after the effects of the drug causing weight-gain, obesity and increase in associated cardio-metabolic risk factors has caused or induced an increase of body weight or associated cardio-metabolic risk factors in the patient. In another embodiment the medicament being manufactured according to the invention is applied after the drug causing weight gain, obesity and increase in associated cardio-metabolic risk factors has been applied, but before or during the onset of the increase in body-weight or associated cardio-metabolic risk factors.
In a further embodiment the medicament being manufactured according to the invention is applied at the same time ( or approximately the same time) when the drug causing weight gain, obesity and increase in associated cardio-metabolic risk factors is applied. Then the application of the drug and the medicament manufactured according to the invention may be done through different pharmceutical pathways, e.g. orally/parentaral, or through the same pharmaceutical pathway. The application of the drug and the medicament manufactured according to the invention may then be done at the same or different sites of application or using distinct or the same pharmaceutical formulation or medicament. Thus, the application of the drug causing weight gain, obesity and increase in associated cardio-metabolic risk factors and of the 5HT6-Ligand used according to the invention in the the manufacture of the medicament may also be done inside the same pharmaceutical formulation.

The following figures and examples are provided to illustrate the claimed invention and are not meant in any way to limit it.

### Figures:

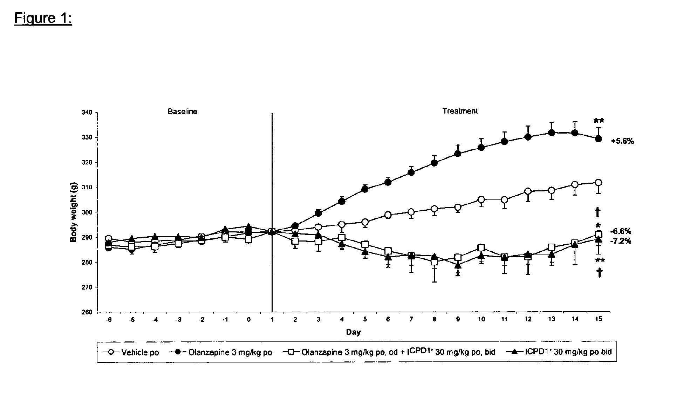

- Figure 1:: Effect of chronic administration of olanzapine and COMPOUND 1, alone and in combination, on body weight in female Sprague-Dawley rats maintained on a high-fat diet. Results are means (adjusted for differences between the body weights of the different treatment groups at baseline (Day 1)) ± SEM (calculated from residuals of the statistical model), n=10. Drug doses are for the free base. Animals were dosed at 0 h and 6 h. Vehicle po = vehicle at 0h and 6h; Olanzapine 3 mg/kg po = olanzapine at 0h and vehicle at 6h; Olanzapine 3 mg/kg po, od + COMPOUND 1 30 mg/kg po, bid = Olanzapine + COMPOUND 1 at 0h and COMPOUND 1 at 6h; COMPOUND 1 30 mg/kg po, bid = COMPOUND 1 at 0h and 6h. Numbers at Day 15 represent % change in body weight compared to the vehicle-treated control group on Day 15 (ie after 14 days of treatment).
Significant differences assessed using Student's t-test: *p<0.02, **p<0.01 vs vehicle, †p<0.001 vs olanzapine.

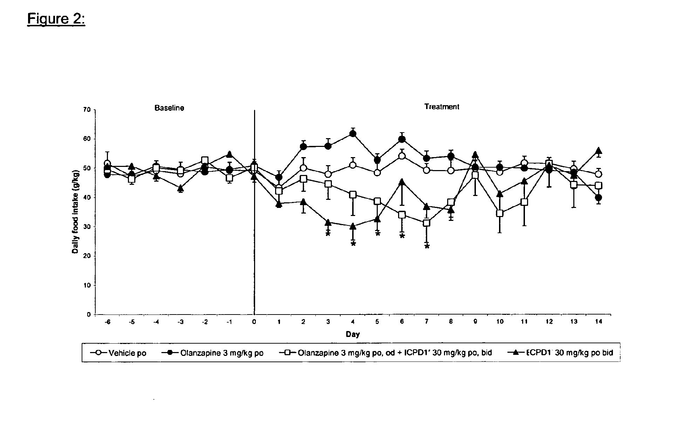

- Figure 2:: Effect of chronic administration of olanzapine and COMPOUND 1, alone and in combination, on daily food intake in female Sprague-Dawley rats maintained on a high-fat diet. Results are means (adjusted for differences between the food intakes of the different treatment groups at baseline [average of Days -6 to 0]) ± SEM (calculated from residuals of the statistical model), n=10. Drug doses are for the free base. Animals were dosed at 0 h and 6 h. Vehicle po = vehicle at 0h and 6h; Olanzapine 3 mg/kg po = olanzapine at 0h and vehicle at 6h; Olanzapine 3 mg/kg po, od + COMPOUND 1 30 mg/kg po, bid = Olanzapine + COMPOUND 1 at 0h and COMPOUND 1 at 6h; COMPOUND 1 30 mg/kg po bid = COMPOUND 1 at 0h and 6h. Multiple comparisons against the vehicle-treated control group were by Dunnett's test. Significant differences from the vehicle-treated group are denoted by *p<0.05 (p levels have only been given at one level for clarity).

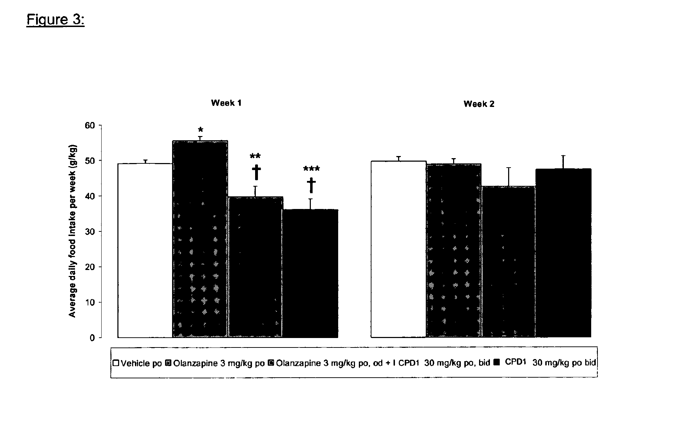

- Figure 3:: Effect of chronic administration of olanzapine and COMPOUND 1, alone and in combination, on average daily food intake per week in female Sprague-Dawley rats maintained on a high-fat diet. Results are means (adjusted for differences between the food intakes of the different treatment groups at baseline [average of Days -6 to 0]) + SEM (calculated from residuals of the statistical model), n=10. Drug doses are for the free base. Animals were dosed at 0 h and 6 h. Vehicle po = vehicle at 0h and 6h; Olanzapine 3 mg/kg po = olanzapine at 0h and vehicle at 6h; Olanzapine 3 mg/kg po, od + COMPOUND 1 30 mg/kg po, bid = Olanzapine + COMPOUND 1 at 0h and COMPOUND 1 at 6h; COMPOUND 1 30 mg/kg po, bid = COMPOUND 1 at 0h and 6h Significant differences from vehicle assessed using Student's t-test: *p<0.05, **p<0.01, ***p<0.001 vs vehicle, †p<0.001 vs olanzapine.

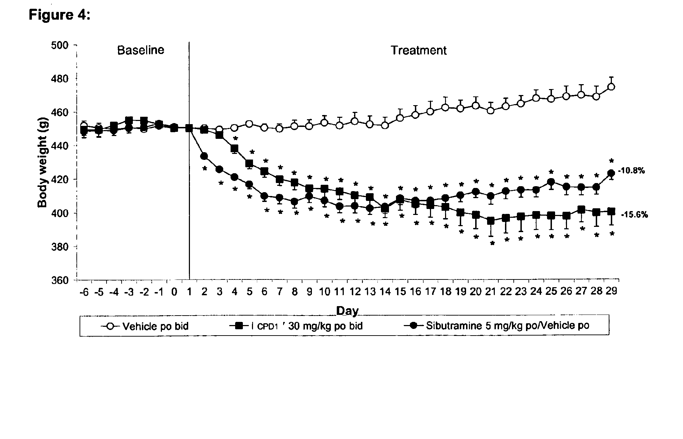

- Figure 4: A comparison of the effects of chronic administration of the 5-HT₆ receptor ligand,COMPOUND 1, and the reference anti-obesity drug, sibutramine, on body weight in dietary-induced obese, female Wistar rats

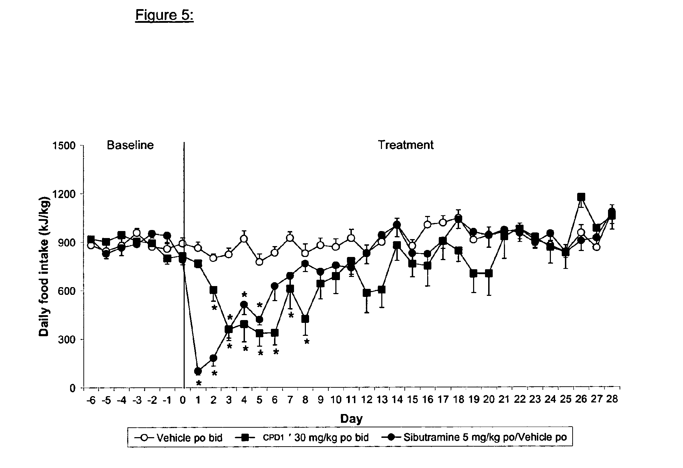

- Figure 5: A comparison of the effects of chronic administration of the 5-HT₆ receptor ligand, COMPOUND1, and the reference anti-obesity drug, sibutramine, on daily food intake in dietary-induced obese, female Wistar rats.Results are means (adjusted for differences between treatment groups at baseline [average of Days -6 to 0]) ± SEM (calculated from the residuals of the statistical model), n=9-10. Multiple comparisons against the vehicle-treated control group were by Dunnett's test. Significant differences from the control group are denoted by *p<0.05 (p levels have only been given at one level for clarity).

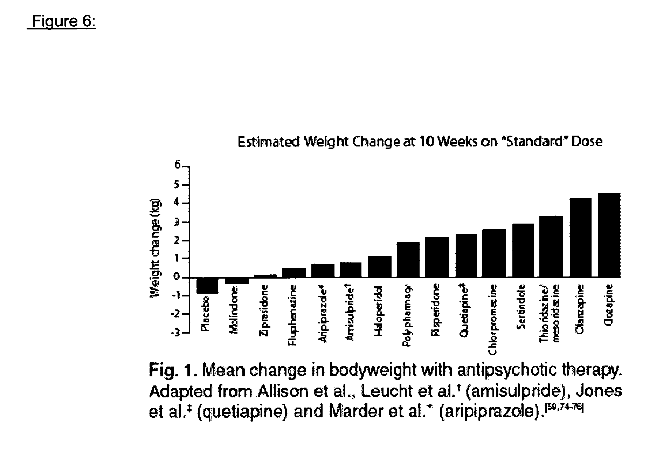

- Figure 6:: The rank order of the actions of various antipsychotic drugs to evoke weight-gain in patients taken from Newcomer (2005). Mean change in body weight with antipsychotic therapy.

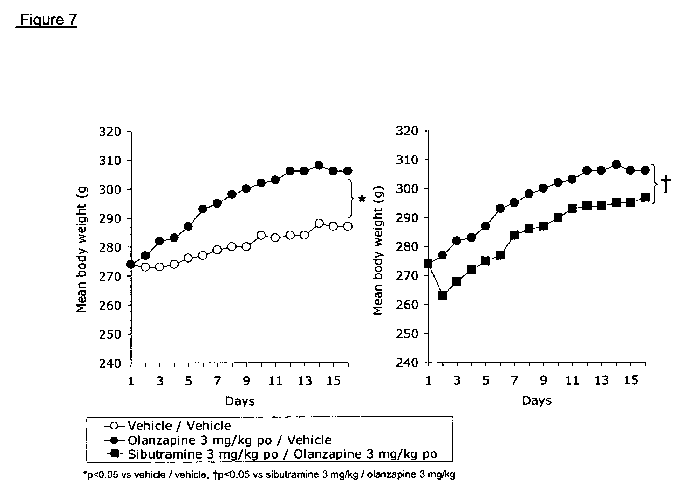

- Figure 7:: The effect of olanzapine to evoke weight gain in rats and the partial prevention of this effect by repeated sibutramine administration taken from Heal & Jagger (2005).

### Examples:

The findings exemplified herein support the following claims.
1. A compound binding to the 5HT6 receptor will be therapeutically beneficial as an adjunctive medicament to treat or prevent the actions of typical and atypical antipsychotic drugs to cause weight-gain, obesity and increase cardio-metabolic risk in the treatment of schizophrenia, related psychiatric disorders and other disorders of the brain that are treatable with typical and atypical antipsychotic drugs in children, adolescents and adults.
2. A compound binding to the 5HT6 receptor will be therapeutically beneficial as an adjunctive medicament to treat or prevent the actions of other drugs known to cause weight-gain, obesity and increase cardio-metabolic risk taken from the list including, but not limited to, antidepressants including MAOIs, TCAs, SSRIs, "third generation" and atypical antidepressants, anti-manic agents, corticosteroids, agents for insulin resistance, impaired glucose tolerance and Type 2 diabetes, antihypertensives, anti-ulceratives, medicaments for neuropathic pain, antihistamines, anti-epileptics, medicaments for migraine, benzodiazepines, contraceptives and hormone replacement therapy, in children, adolescents and adults, who are in need of treatment with one or more of the medicaments described in the non-limiting list above.

For exemplifying this effect olanzapine was used as the therapeutical drug. Olanzapine is known to cause weight-gain, obesity and associated cardio-metabolic risk. It is an atypical antipsychotic and is used here as a representative neuroleptic that causes weight-gain, obesity and related metabolic disturbances.

The compound binding to the 5HT6 receptor used was 5-Chloro-naphthalene-2-sulfonic acid [3-(2-dimethylamino-ethyl)-1H-indol-5-yl]-amide (hereinafter called COMPOUND 1):

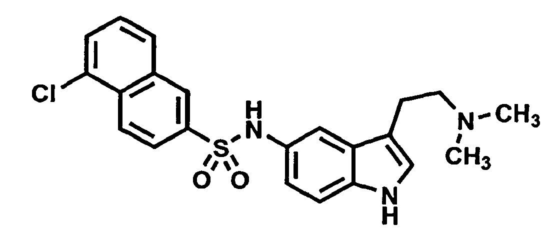

### Example 1:

### Radiolabelled ligand-receptor binding experiments performed on cloned/transfected human 5-HT₆ receptors in vitro

### Example 1 Determination of the affinity of COMPOUND 1 for cloned/transfected human 5-HT₆ receptors.

### METHODS

To investigate the binding properties of 5-HT₆ receptor ligands to human 5-HT₆ receptors, transfected HEK-293 membranes from Perkin-Elmer and [3H]-LSD (NEN), as the radioligand, were used. The assay was carried out in 96-well plates with a total reaction volume of 200 µl, containing 100 µl of membrane suspension (35 µg protein/well), 10 µl of [3H]-LSD (2.7 nM) in either the absence or presence of 90 µl of either buffer or methiothepin (5 µM) for total and non-specific binding, respectively. Binding buffer contained 50 mMTris-HCl, 10 mM MgCl₂ and 0.5 mM EDTA at pH 7.4. Plates were incubated at 37 °C for 60 minutes. After the incubation period, 170 µl of incubate were transferred to MultiScreen HTS, FC plates (Millipore), filtered and plates were washed 3 times with ice-cold 50mM Tris-HCl (pH 7.4). Filters were dried and counted at approximately 40% efficiency in a MicroBeta scintillation counter (Perkin-Elmer) using 25 µl per well of EcoScint liquid scintillation cocktail.

### RESULTS

When the affinity of COMPOUND 1 for cloned human 5-HT₆ receptors stably transfected and expressed in HEK-293 cells was tested, this compound was observed to bind with high affinity to this serotonergic receptor subtype having a pKi value of 9.13 ± 0.17 (n ≥ 6) equating to a Ki of 0.74 ηM.

### Experiments performed in rats in vivo to determine the effects of 5-HT₆ ligands on neuroleptic-induced weight-gain

### Example 2 Effect of repeated administration of COMPOUND 1 on body weight-gain and hyperphagia evoked in rats by the atypical antipsychotic drug, olanzapine.

### METHODS

### Animals

The experiment was performed on 40 female Sprague-Dawley rats (originally in the weight range 250-300 g). The animal room was maintained at a temperature of 21±4°C and 55±20% humidity and on a reversed phase light-dark cycle (lights off for 8 h from 09.30-17.30 h) during which time the room was illuminated by red light. Animals had free access to powdered high fat diet (VRF1 plus 20% lard). The diet was contained in a glass feeding jar with an aluminium lid with a 3-4 cm hole cut in it to allow access to the food. Animals were housed singly in polypropylene cages with wire grid floors to enable the food intake of each rat to be recorded. Polypropylene trays containing cage pads were placed beneath each cage to detect any food spillage. Animals were accustomed to these new conditions (individual housing and the wire-grid floor cages) for at least 14 days before the start of the baseline readings.

### Experimental procedures for the feeding study

Approximately a week before the start of the study, animals were weighed (to the nearest 0.1 g using an electronic top-pan balance) and allocated into 4 weight-matched treatment groups, each containing 10 animals. Following a 7 day baseline run-in period, during which the animals were dosed orally with vehicle (0.5% hydroxypropylmethylcellulose), to acclimatise them to dosing/handling, the rats were given treatments, described in the Table below, for 14 days.

| | | |
|---|---|---|
| Treatment 1 (vehicle/drugs given in one solution at 0 h) | Treatment 2 (6 h) | n |
| Vehicle po | Vehicle po | 10 |
| Olanzapine (3 mg/kg po) | Vehicle po | 10 |
| Olanzapine (3 mg/kg po) + COMPOUND 1 (30 mg/kg po) | COMPOUND 1 (30 mg/kg po) | 10 |
| COMPOUND 1 (30 mg/kg po) | COMPOUND 1 (30 mg/kg po) | 10 |

Food intake and body weight were measured once daily. Variations in body weight were accounted for by expressing the food intake results in terms of g/kg rat weight.

Statistical comparisons between the body weights of the different treatment groups were made by analysis of covariance followed by multiple t tests (two-tailed) to compare each treatment group with the vehicle-treated controls. For the body weight data (body weights recorded each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

Statistical comparisons between the food intakes of the different treatment groups were made by analysis of covariance followed by multiple t tests (two-tailed) to compare each treatment group with the vehicle-treated controls. Food intake of the animals at baseline (ie average of Day -6 to Day 0) was used as the covariate. Standard errors of the mean (SEM) were calculated from the residuals of the statistical model.

Analysis of body weights and average daily food intakes were also performed to analyse cumulative food intake and body weight changes. Analysis of covariance followed by post-hoc Student's t-test (two-tailed) was used to compare the average daily food intakes over Week 1 and Week 2 for COMPOUND 1 treatment group versus (a) the vehicle-treated and (b) the olanzapine-treated groups of rats. The body weights of all groups of rats before and after treatment were statistically compared pair-wise using Student's paired t-test (two-tailed).

### RESULTS

### Effects of COMPOUND 1 on bodyweight and on olanzapine-induced weight-gain in female Sprague-Dawley rats

As shown in Figure 1, when olanzapine (3 mg/kg po) + vehicle was administered daily for a period of 14 days to female, Sprague-Dawley rats that were given access to a high-fat diet, it evoked a marked and sustained increase in the body weights of these rats when compared to the vehicle + vehicle-treated control (Figure 1). On Day 15, the mean weight of the olanzapine (3 mg/kg po) + vehicle-treated group of rats was statistically (p < 0.01) greater than that of the vehicle + vehicle-treated controls (Figure 1). In contrast, the group of rats given COMPOUND 1 (30 mg/kg po bid) showed a gradual decrease in body weight compared to the vehicle + vehicle-treated controls that was prolonged and sustained (Figure 1). On Day 15, the mean weight of the COMPOUND 1 (30 mg/kg po bid)-treated group of rats was statistically (p < 0.01) lower than that of the vehicle + vehicle-treated controls (Figure 1). When rats were orally administered the 30 mg/kg bid of COMPOUND 1, it not only prevented the weight-gain evoked by 3 mg/kg of olanzapine, but it also surprisingly reduced the body weights of the rats to less than those treated with vehicle, and in fact, to the same degree as those treated with COMPOUND 1 (30 mg/kg po bid) only (Figure 1). On Day 15, the mean decrease in the body weights of the rats treated with COMPOUND 1 (30 mg/kg po bid) + olanzapine (3 mg/kg po) was statistically lower than the groups treated with olanzapine (3 mg/kg po) + vehicle (p < 0.001), or vehicle + vehicle (p < 0.02) and was not statistically different from those of the group of rats administered COMPOUND 1 (30 mg/kg po bid) (Figure 1).

A paired statistical comparison was made of the body weights of all of the groups of rats at Day 1 versus Day 15. This analysis, reported in Table 1, revealed that the vehicle + vehicle and olanzapine (3 mg/kg po) + vehicle groups of rats experienced a significant (p < 0.001) increase in body weight over the duration of the experiment, but those given COMPOUND 1 (30 mg/kg po, bid) or COMPOUND 1 (30 mg/kg po, bid) + olanzapine (3 mg/kg po) showed no significant accumulation of weight over the same time period.

### Effects of COMPOUND 1 on food intake and on olanzapine-induced hyperphagia in female Sprague-Dawley rats

As shown in Figure 2, when olanzapine (3 mg/kg po) + vehicle was administered daily for a period of 14 days to female, Sprague-Dawley rats which were given access to a high-fat diet, it evoked an increase in food intake when compared to the vehicle-treated controls. The increase in daily food intake was particularly noticeable from Day 2 to Day 7 of olanzapine + vehicle administration (Figure 2). The cumulative intake of food by the olanzapine (3 mg/kg po) + vehicle-treated group of rats was statistically greater (p < 0.05) than that of the vehicle controls over the first week of the experiment (Figure 3). In contrast the group of rats given COMPOUND 1 (30 mg/kg po bid) + vehicle markedly reduced their food intakes compared to the vehicle + vehicle-treated controls (Figure 2). The cumulative intake of food by the COMPOUND 1 (30 mg/kg po bid)-treated group of rats was statistically lower (p < 0.001) than that of the vehicle controls over the first week of the experiment (Figure 3). When rats were orally administered 30 mg/kg bid of COMPOUND 1, it not only prevented the hyperphagia evoked by 3 mg/kg of olanzapine, but it also surprisingly reduced the food intake of the rats to less than those of the vehicle + vehicle controls, and in fact, to the same degree as those treated only with COMPOUND 1 (30 mg/kg po bid) (Figure 3). The decrease in cumulative food consumption over the first week of administration with COMPOUND 1 (30 mg/kg po bid) + olanzapine (3 mg/kg po) was statistically lower than that of the groups of rats treated with olanzapine (3 mg/kg po) + vehicle (p < 0.001) or vehicle + vehicle (p < 0.01) and was not statistically different from that of the group of rats administered COMPOUND 1 (30 mg/kg po bid) (Figure 3).

### Experiments performed in rats in vivo to determine the comparative efficacy of 5-HT₆ ligands and the reference anti-obesity drug, sibutramine, in dietary-induced obese (DIO) rats

### Example 3 A comparison of the effects of COMPOUND 1 and the reference anti-obesity drug, sibutramine, on bodyweight and food intake in female Wistar rats that have become obese through the excessive consumption of highly palatable food sources

### Animals

The experiment was performed on female Wistar rats (originally in the weight range 250-300 g). The rats were housed in pairs in polypropylene cages with solid floors and sawdust bedding at a temperature of 21±4°C and 55±20% humidity. Animals were maintained on a reverse phase light-dark cycle (lights off for 8 h from 09.30-17.30 h) during which time the room was illuminated by red light. Animals had free access to powdered high fat diet (VRF1 plus 20% lard), ground chocolate, ground peanuts and tap water at all times. The three different diets were contained in separate glass feeding jars with aluminum lids with a 3-4 cm hole cut in them to allow access to the food. Animals were housed in pairs for twelve weeks for the induction of obesity. Animals were then housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

### Experimental procedures for the feeding study

At the start of the first DIO study, animals were weighed (to the nearest 0.1 g using an electronic top-pan balance) and allocated into 3 weight-matched treatment groups, each containing 10 animals. Following a 7 day baseline run-in period, during which time all animals were dosed orally once a day with vehicle (0.5% hydroxypropylmethylcellulose), rats were dosed for 28 days with vehicle at 0 and 6 h or test compound either COMPOUND 1 (30 mg/kg, p.o., twice a day at 0 and 6 h) or sibutramine (5 mg/kg, p.o. at 0 h / vehicle p.o. at 6 h). The first compound dose was given at the onset of the 8 h dark period (0 h). This strategy was taken to maximise the impact of any inhibitory effect of the compounds on food intake as rats are predominantly nocturnal feeders. Six hours later, animals were dosed again with either vehicle or compounds. The dosing schedule is shown in the Table below.

| | | |
|---|---|---|
| Treatment 1 (0 h) | Treatment 2 (6 h) | n |
| Vehicle po | Vehicle po | 10 |
| COMPOUND 1 (30 mg/kg po) | COMPOUND 1 (30 mg/kg po) | 10 |
| Sibutramine (5 mg/kg, p.o.) | Vehicle po | 10 |

Food intake and body weight were measured once daily. Variations in body weight and energy levels of the different types of food were accounted for by expressing the food intake results in terms of kJ/kg rat weight.

Statistical comparisons between the body weights and daily food intakes of the different treatment groups have been made by analysis of covariance followed by Dunnett's test to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

### RESULTS

### Effects of COMPOUND 1 and sibutramine on bodyweight in female, DIO, Wistar rats

As shown in Figure 4, when COMPOUND 1 (30 mg/kg po, bid) was administered to the female, cafeteria-fed, DIO Wistar rats for a period of 28 days, it evoked a marked and sustained decrease in the body weights of these obese rats. The body weights of the COMPOUND 1-treated rats were statistically reduced compared with the vehicle-treated controls on Day 2 of treatment and they remained significantly lower than those of the controls throughout the 28 day duration of COMPOUND 1 treatment.

Repeated administration of reference anti-obesity drug, sibutramine (5 mg/kg/day, p.o., once daily), also induced significant body weight-loss in female, DIO, Wistar rats compared with the vehicle-treated animals during the 4-week dosing period (Figure 4). The weight-loss evoked by sibutramine was more rapid in onset than that caused by COMPOUND 1 being statistically significant from Day 1 onwards, but the body weight curves crossed at Day 15, with COMPOUND 1-treated rats continuing to lose more weight than the sibutramine group before the plateau was reached. t the end of treatment period (Day 28) the rats treated with COMPOUND 1 and sibutramine weighed 15.6 % and 10.8 % less than the vehicle-treated controls, respectively.

### Effects of COMPOUND 1 and sibutramine on food intake in female, Wistar, DIO rats

As shown in Figure 5, when COMPOUND 1 (30 mg/kg po, bid) was administered to the female, cafeteria-fed, DIO Wistar rats for a period of 28 days, it produced a reduction in daily food intake that was apparent over the first 10 days of treatment. These decreases were statistically significant versus the vehicle-treated control group from Day 2 to Day 8.

Repeated administration of reference anti-obesity drug, sibutramine (5 mg/kg/day, p.o., once daily), also produced a significant reduction of food intake the initial 13 days of treatment (Figure 5). However, the onset of action of this drug on food intake after the start of drug administration was more rapid than observed with COMPOUND 1. These decreases were statistically significant versus the vehicle-treated control group from Day 1 to Day 5 of the experiment.

For both of the drugs, food intake had returned to the same level the controls by Day 14 (Figure 5).

### DISCUSSION

Weight-gain as a side-effect is observed with drugs from many different pharmacological and therapeutic classes. However in most instances, it is both an unwanted effect and one that has adverse health consequences by predisposing the patient to an increased risk of dyslipidaemia, insulin resistance, impaired glucose tolerance, Type 2 diabetes, Metabolic Syndrome and/or a cardio- or cerebrovascular accident. Moreover, unwanted weight-gain can be a major factor in patients' non-compliance to drug therapy.

When discussing the atypical antipsychotic drugs, it has been reported that these drugs cause deleterious increases in the plasma concentrations of triglycerides and glucose (Wirshing et al, 2002; Casey 2004; see also the reviews of Newcomer, 2005 and Melkersson & Dahl, 2004), and this is in a vulnerable patient population who already have increased visceral adiposity compared with normal subjects (Ryan et al, 2004).

The findings reported here show that a compound binding to the 5HT6 receptor, as exemplified by COMPOUND 1, prevented the weight-gain that was evoked by an antipsychotic drug, exemplified by olanzapine. Furthermore, olanzapine is a drug that is known not only to be one of the worst offenders for producing weight-gain as a side-effect in this class of drugs (Newcomer, 2005; Figure 6), but also amongst all drugs that cause weight increase (Pijl & Meinders, 1996). Unexpectedly, COMPOUND 1 was found not only to abolish olanzapine-induced weight-gain, but actually to produce weight-loss in this group of rats in comparison to the vehicle-treated controls which had been given access to a high-fat diet.

These findings were unexpected for three reasons; first because this 5-HT₆ ligand abolished neuroleptic-induced weight gain, second because it evoked a weight-loss, and third, because the ability of drugs to evoke clinically significant weight-loss in animal models of obesity is not predictive of efficacy in the treatment of neuroleptic-induced weight-gain. This point is demonstrated by the results presented in **Example 3** and by data taken from Heal & Jagger (2005) shown in Figure 7. Thus, Figure 4 clearly shows that the 5-HT₆ receptor ligand, COMPOUND 1, and the reference comparator, the serotonin and noradrenaline reuptake inhibitor (SNRI) anti-obesity drug, sibutramine (Meridia®, Reductil®), both evoke profound weight reduction in the DIO rat, which is an excellent model of human obesity (Heal & Jagger, 2005). In both cases, the weight reductions were greater than 10% over the 28 daily treatment period and the reductions were maintained throughout the duration of the drug treatment, ie there was no drug tolerance (Figure 4). As shown by the data in Figure 5, the decreases of bodyweight in both cases were concomitant with a reduction of food consumption. Thus, sibutramine, which evokes a greater suppression of food intake on initiation of treatment, produced a more rapid fall in bodyweight than COMPOUND 1. Overall, the 5-HT₆ ligand and sibutramine both produced clinically significant weight-loss by a pharmacologically relevant mechanism, ie a reduction of food intake. In spite of this fact, the data reported in **Example 3** and presented in Figure 1 show that although the 5-HT₆ ligand abolished olanzapine-induced weight-gain and evoked weight-loss versus the vehicle control, sibutramine was able only partially to attenuate the weight-gain evoked by this atypical antipsychotic (Figure 7). Of particular note is the fact that the experiments performed to investigate the prevention of neuroleptic weight-gain shown in Figures 1, 2 and 7 were performed using a drug treatment schedule of 14 days, and in Figure 4, it shows that the weight-losses evoked by sibutramine and COMPOUND 1 were identical at Days 14 and 15, thereby confirming that anti-obesity efficacy per se does not predict an ability to prevent neuroleptic-induced weight-gain.

The degree of weight-loss observed in the rats administered COMPOUND 1 + olanzapine was not significantly different from that observed when COMPOUND 1 was given alone, indicating that the therapeutic benefit of this pharmacological modality was not attenuated by combination with that of an antipsychotic drug. In defining the pharmacological mechanisms responsible for these effects, it is evident that the weight-gain observed with olanzapine treatment was caused by an increase in food consumption and the action of COMPOUND 1 to prevent this weight-gain and to cause weight-loss was due to a reduction in food intake to a level that was significantly lower than that of the vehicle-treated control group. In short, these observations demonstrate a clinically acceptable mechanism for the prevention of antipsychotic-induced weight-gain and to cause weight-loss.

Here we have used COMPOUND 1 as an example of a 5-HT₆ receptor ligand. Although COMPOUND 1 has been reported to be an agonist of the rat and human variants of the 5-HT₆ receptor (Romero et al, 2006), the findings reported here can be extended to all 5-HT₆ receptor ligands, irrespective of their functionality, for the following reasons:
1. It has been reported that paradoxically both agonist-like 5-HT₆ ligands, eg COMPOUND 1 (Figures 1 and 2) and antagonists, eg BVT 5182 and PRX-07034 (Svartengren et al, 2004; Shacham et al, 2006), have the ability to reduce food intake and produce sustained weight-loss in obese rodents
2. All of the pharmacological characterisation of agonist/antagonist functionality on rat or human 5-HT₆ receptor subtypes is based on experiments performed using cloned receptors expressed in cell-lines, eg a human embryonic kidney cell-line or Chinese hamster ovary cells, not 5-HT₆ receptors present in their native environment in the physiologically relevant tissues, ie the brain. As previously demonstrated by Hoyer & Boddeke (1993), data from the former are exquisitely sensitive to variations in the test systems employed. Thus, the selected cell-line, receptor density and the G-protein combinations employed in the cell-based system can result in different functional outcomes when individual drugs are tested; yielding functionality predictions for a given drug ranging from antagonism through to full agonism at the same receptor. Thus, agonist/antagonist functionality predictions based on results from in vitro experiments using cloned receptors expressed in cell-lines cannot be taken as definitive and are applicable only to the artificial test system in which the data were generated.

The treatment or prevention of neuroleptic-induced weight-gain by administering to a patient a medicament containing a compound binding to the 5HT& receptor will provide clinical benefit to the patient by avoiding deleterious changes in obesity-related cardio-metabolic risk factors, ie increased visceral adiposity and/or increases in plasma triglycerides, cholesterol and/or LDL-cholesterol, decreases in HDL-cholesterol, insulin resistance and/or impaired glucose tolerance and/or hypertension that predisposes him/her to a greatly increased risk of developing dyslipidaemia and/or Type 2 diabetes and/or Metabolic Syndrome, or of suffering a cardio- or cerebrovascular accident.

In addition, the weight-loss produced in a patient by administration of a medicament containing a **5-HT₆ ligand** will be of especial therapeutic benefit to a patient, who is in need of therapy with a typical or atypical antipsychotic drug that causes weight-gain, and who is overweight or obese and/or metabolically compromised, and as a consequence, is at increased cardio-metabolic risk. The weight-loss will improve various obesity-related cardio-metabolic risk factors, ie increased visceral adiposity and/or increased plasma triglycerides, cholesterol and/or LDL-cholesterol, decreases in HDL-cholesterol, insulin resistance and/or impaired glucose tolerance and/or hypertension that predispose him/her to a greatly increased risk of developing dyslipidaemia and/or Type 2 diabetes and/or Metabolic Syndrome, or of suffering a cardio- or cerebrovascular accident.

The finding that the weight-loss produced by treatment with COMPOUND 1 + olanzapine was not different from that evoked COMPOUND 1 alone clearly demonstrates that the pharmacological actions of olanzapine do not reduce the cardio-metabolic benefits of COMPOUND 1 treatment. It is well known that many, but by no means all, typical and atypical antipsychotic drugs have affinity for CNS 5-HT₆ receptors (Roth et al, 1994). In the case of olanzapine, which has been used in these Examples because it evokes profound weight-gain in man, this drug is one of the atypical antipsychotics which has a very high affinity for the human 5-HT₆ receptor (Roth et al, 1994; Bymaster et al, 1999). However, the role of 5-HT₆ receptor mechanisms in the therapeutic profile of the antipsychotics remains enigmatic with efficacy in treating schizophrenia and related psychiatric disorders being generally believed to derive from their antagonist actions at dopamine D2 and 5-HT_{2A} receptors (Meltzer, 1999). It is known that there are marked similarities between the distribution of the 5-HT₆ receptor in the brains of humans and rats (East et al, 2002). Furthermore, it has been reported that the density of 5-HT₆ receptors is not altered in the brains of schizophrenics, nor is their number in the brains of rats altered after chronic antipsychotic treatment (East et al, 2002); both findings are consistent with the view set out by Meltzer (1999) suggesting this serotonergic receptor subtype is not a primary target for antipsychotic drug action. The observation, therefore, that COMPOUND 1 not only prevents the weight-gain produced by olanzapine, which itself has high 5-HT₆ receptor affinity, demonstrates that this pharmacological property of the antipsychotic drug does not attenuate the weight-loss and associated cardio-metabolic benefits of **5-HT₆ ligands .**

Viewing the pharmacological action of **5-HT₆ ligands** from the perspective of potentially modifying the therapeutic benefits provided by a typical or atypical antipsychotic drug, it is known that the actions of such drugs on 5-HT₆ receptors are not a prerequisite for efficacy in the treatment of schizophrenia and related psychiatric disorders. The rationale being that antipsychotic drugs like raclopride, rimcazole and penfluridol, which are efficacious in the clinic, have no relevant affinity for this 5-HT receptor subtype (Roth et al, 1994). Thus, the pharmacological action of the 5-HT₆ receptor ligand is predicted to have no attenuating effect on the efficacy of typical or atypical antipsychotic drugs. Furthermore, it has been postulated that 5-HT₆ receptor ligands are likely to deliver benefits in the treatment of schizophrenia and related psychiatric disorders because they have cognitive enhancing properties (see reviews by Mitchell & Neumaier, 2005 and Holenz et al, 2006) and reversing cognitive impairment is a major unmet clinical need in the treatment of schizophrenia, particularly in treating the negative symptoms of schizophrenia (Mohs, 1999; Mohamed et al, 1999; Harvey et al, 2006).

Olanzapine was selected as an **Example** not only because it is an atypical antipsychotic drug that is known to be one of the worst offenders in this class of drugs for producing weight-gain (Newcomer, 2005; Melkersson & Dahl, 2004), but also because it is amongst the worst of all drugs that cause weight increase (Pijl; & Meinders, 1996). As such, we have used olanzapine as a generic **Example** of any drug that causes weight-gain/obesity as a side-effect when evaluating the beneficial actions of **5-HT₈ ligands** to prevent drug-induced weight-gain and to produce weight-loss. Examples of other drugs causing weight-gain/obesity and increased cardio-metabolic risk include, but are not limited to, antidepressants including MAOls, TCAs, SSRls, "third generation" and atypical antidepressants, anti-manic agents, corticosteroids, medicaments for the treatment of insulin resistance, impaired glucose tolerance and Type 2 diabetes, antihypertensives, anti-ulceratives, anti-epileptics, medicaments for treating neuropathic pain, antihistamines, medicaments for treating migraine, benzodiazepines, female hormonal contraceptives and female hormone replacement therapy.

In a second aspect of the invention, the use is claimed of **5-HT₆ ligands** as adjunctive medicaments to treat or prevent the weight-gain, obesity and cardio-metabolic consequences caused by administration to a patient in need of treatment with a drug taken from the list including, but not limited to, antidepressants including MAOls, TCAs, SSRls, "third generation" and atypical antidepressants, anti-manic agents, corticosteroids, agents for insulin resistance, impaired glucose tolerance and Type 2 diabetes, antihypertensives, anti-gastric ulceratives, anti-epileptics, medicaments for neuropathic pain, antihistamines, medicaments for migraine, benzodiazepines, contraceptives and hormone replacement therapy. Drug-induced obesity, or any increase of adiposity is unacceptable in patients who have metabolic disturbances associated with increased cardiac risk. As an example, weight gain induced by anti-diabetic drugs, eg the sulphonylureas, thiazolidinediones and/or insulin therapy, in treating insulin-resistance, impaired glucose tolerance and/or Type 2 diabetes is contra-indicated because of the deleterious effect that adiposity has on glycaemic control. In such cases, not only is increased weight not an acceptable outcome, but weight-loss will be in many instances of considerable therapeutic benefit to the patients concerned.

None of the drugs, which have been selected from the following therapeutic classes that are known to produce weight-gain as a side-effect including, but not limited to, antidepressants including MAOls, TCAs, SSRls, "third generation" and atypical antidepressants, anti-manic agents, corticosteroids, agents for insulin resistance, impaired glucose tolerance and Type 2 diabetes, antihypertensives, anti-ulceratives, anti-epileptics, agents for neuropathic pain, antihistamines, agents for migraine, benzodiazepines, female hormonal contraceptives and female hormone replacement therapy, produce their therapeutic actions via actions on CNS 5-HT₆ receptors. Hence, these drugs are not predicted to reduce the therapeutic benefit of a **5-HT₆ ligand,** as exemplified by COMPOUND 1, to treat or prevent weight-gain, obesity and associated cardio-metabolic risk. Even if the pharmacological profile of such a weight-enhancing drug did include 5-HT₆ receptor affininity and it was capable of crossing the blood-brain barrier, the failure of the brain penetrating 5-HT₆ antagonist, olanzapine, to attenuate the therapeutic benefits of COMPOUND 1 demonstrate that such an effect will not detract from the clinical benefits provided by **5-HT₆ ligands,**

Conversely, because none of the drugs, which have been selected from the following therapeutic classes that are known to produce weight-gain as a side-effect including, but not limited to, antidepressants including MAOls, TCAs, SSRls, "third generation" and atypical antidepressants, anti-manic agents, corticosteroids, agents for insulin resistance, impaired glucose tolerance and Type 2 diabetes, antihypertensives, anti-ulceratives, anti-epileptics, agents for neuropathic pain, antihistamines, agents for migraine, benzodiazepines, female hormonal contraceptives and female hormone replacement therapy, produce their therapeutic effects via actions on CNS 5-HT₆ receptors, it can be concluded that the administration of a an adjunctive medicament treat or prevent weight-gain, obesity and associated cardio-metabolic risk containing a **5-HT₆** ligand to will not detract from the clinical benefits provided by the drugs described in the non-limiting list above.

Hence, the results can be extended also to support a claim for the use of **5-HT₆ ligands** for the treatment or prevention of weight-gain, obesity and associated cardio-metabolic risk factors induced by other drugs selected from the following therapeutic classes including, but not limited to, antidepressants including MAOls, TCAs, SSRls, "third generation" and atypical antidepressants, anti-manic agents, corticosteroids, medicaments for the treatment of insulin resistance, impaired glucose tolerance and Type 2 diabetes, antihypertensives, anti-ulceratives, anti-epileptics, medicaments for treating neuropathic pain, antihistamines, medicaments for treating migraine, benzodiazepines, female hormonal contraceptives and female hormone replacement therapy.

### REFERENCES

American Heart Association. Third Report of the National Cholesterol Education Program (NCEP) Expert Panel on Detection Evaluation, and Treatment of High Blood Cholesterol in Adults (Adult Treatment Panel III) Finial Report. Circulation 2002;106:3143-3420.
Bymaster F, Perry KW, Nelson DL, Wong DT, Rasmussen K, Moore NA, Calligaro DO. Olanzapine: a basic science update. Br J Psychiatry Suppl. 1999:36-40.
Casey DE. Dyslipidemia and atypical antipsychotic drugs. J Clin Psychiatry. 2004;65 Suppl 18:27-35.
East SZ, Burnet PW, Leslie RA, Roberts JC, Harrison PJ. 5-HT6 receptor binding sites in schizophrenia and following antipsychotic drug administration: autoradiographic studies with [125I]SB-258585. Synapse. 2002;45:191-9.
Haapasalo-Pesu KM, Saarijarvi S. Olanzapine induces remarkable weight gain in adolescent patients. Eur Child Adolesc Psychiatry. 2001;10:205-8.
Harvey PD, Koren D, Reichenberg A, Bowie CR. Negative symptoms and cognitive deficits: what is the nature of their relationship? Schizophr Bull. 2006;32:250-8.
Heal DJ & Jagger E. Development, regulatory and marketing challenges for novel anti-obesity therapies. In: Obesity and Metabolic Disorders. Eds Antel J, Finer N, Heal DJ, Krause G.IOS Press, Amsterdam. 2005, pp73-91.
Holenz J, Pauwels PJ, Diaz JL, Merce R, Codony X, Buschmann H. Medicinal chemistry strategies to 5-HT(6) receptor ligands as potential cognitive enhancers and antiobesity agents. Drug Discov Today. 2006; 11:283-99.
IDF consensus worldwide definition of the Metabolic Syndrome. IDF 2005. www.idf.org. Melkersson K, Dahl M-L. Adverse metabolic effects associated with atypical antipsychotics. Literature review and clinical implications. Drugs. 2004;64:701-23.
Meltzer HY. The role of serotonin in antipsychotic drug action. Neuropsychopharmacology. 1999;21(Suppl):106S-115S.
Mitchell ES, Neumaier JF. 5-HT6 receptors: a novel target for cognitive enhancement. Pharmacol Ther. 2005;108:320-33.
Mohamed S, Paulsen JS, O'Leary D, Arndt S, Andreasen N. Generalized cognitive deficits in schizophrenia: a study of first-episode patients. Arch Gen Psychiatry. 1999;56:749-54.
Mohs RC. Cognition in schizophrenia: natural history, assessment and clinical importance. Neuropsychopharmacol. 1999;21:S203-S210.
Newcomer JW. Second-generation (atypical) antipsychotics and metabolic effects: a comprehensive literature review. CNS Drugs. 2005;19 Suppl 1:1-93.
Pijl H, Meinders AE. Bodyweight change as an adverse effect of drug treatment. Mechanisms and management. Drug Saf. 1996;14:329-42.
Ratzoni G, Gothelf D, Brand-Gothelf A, Reidman J, Kikinzon L, Gal G, Phillip M, Apter A, Weizman R. Weight gain associated with olanzapine and risperidone in adolescent patients: a comparative prospective study. J Am Acad Child Adolesc Psychiatry. 2002;41:337-43.
Roth BL, Craigo SC, Choudhary MS, Uluer A, Monsma FJ Jr, Shen Y, Meltzer HY, Sibley DR. Binding of typical and atypical antipsychotic agents to 5-hydroxytryptamine-6 and 5-hydroxytryptamine-7 receptors. J Pharmacol Exp Ther. 1994;268:1403-10.
Ryan MC, Flanagan S, Kinsella U, Keeling F, Thakore JH. The effects of atypical antipsychotics on visceral fat distribution in first episode, drug-naive patients with schizophrenia. Life Sci. 2004;74:1999-2008.
WHO: World Health Organization. Definition, diagnosis and classification of diabetes mellitus and its complications. Report of a WHO consultation 1999.
Wirshing DA, Boyd JA, Meng LR, Ballon JS, Marder SR, Wirshing WC. The effects of novel antipsychotics on glucose and lipid levels. J Clin Psychiatry. 2002;63:856-65.

**Table 1 An analysis of the bodyweights at the start and the end of the experiment to determine the effect of COMPOUND 1 on olanzapine-induced weight-gain showing no significant increase of body weight in the group of rats treated with the combination of olanzapine (3 mg/kg po) + COMPOUND 1 (30 mg/kg po, bid)**

| Treatment | Mean body weight (g) Day 0 | Mean body weight (g) Day 15 | Significance |
|---|---|---|---|
| Vehicle + vehicle | 291.9 ± 5.7 | 311.3 ± 8.5 | P<0.001 |
| Olanzapine (3 mg/kg po) + vehicle | 296.9 ± 7.2 | 333.0 ± 8.2 | P<0.001 |
| Olanzapine (3 mg/kg po) + COMPOUND 1 (30 mg/kg po, bid) | 293.4 ± 10.0 | 292.0 ± 10.1 | NS |
| COMPOUND 1 (30 mg/kg po, bid) + vehicle | 292.8 ± 7.2 | 289.5 ± 6.1 | NS |

The bodyweights of the rats before (Day 0) and after treatment (Day 15) were statistically analysed using Student's paired t-test.

## Claims

1. Use of a compound binding to the 5HT6-receptor in the manufacture of a medicament for the treatment of weight-gain, obesity and associated cardio-metabolic risk factors induced by a therapeutic drug.

2. Use according to claim 1, wherein the compound binding to the 5HT6-receptor is selected from sulfonamide compounds according to formula I, 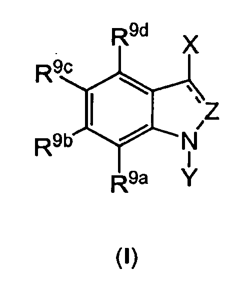 **wherein**
**--Z** either represents =C(R¹⁰) or -CH₂ or =N;
**and wherein**
**either**
**Y** represents -S(O₂)-R¹³, while X represents R¹;
or
**X** represents R¹, while **Y** represents (CH₂)ₙ-R¹¹ or
**Y** represents R¹, while **X** represents (CH₂)ₙ-R¹²;
while
one of R^{9a}, R^{9b}, R^{9c}, or R^{9d} represents N(R³)-S(O₂)-A, or N-(S(O₂)-A)₂;
**and wherein**
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH,-C(=O)-C₁₋₅-alkyl, C(=O)-O-C₁₋₅-alkyl, -O-C(=O)-C₁₋₅-alkyl, oxo (=O), F, Cl, Br, I, -CN,-OCF₃, -SCF₃, -OH, -SH, -NH₂, -NH(C₁₋₅-alkyl), -N(C₁₋₅-alkyl)₂, -NH-C(=O)-C₁₋₅-alkyl,-N(C₁₋₅-alkyl)-C(=O)-C₁₋₅-alkyl, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy, benzyl, and pyridinyl and which may be condensed with a saturated or unsaturated mono- or bicyclic ring system, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S),-C(=O)-OH, -C(=O)-O-C₁₋₅-alkyl, -O-C(=O)-C₁₋₅-alkyl, F, Cl, Br, I, -CN, -CF₃, -OCF₃,-SCF₃, -OH, -SH, -NH₂, -NH(C₁₋₅-alkyl), -N(C₁₋₅-alkyl)₂, -NO₂, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, -OH,-NH₂, -SH, -O-CH₃, -O-C₂H₅, -NO₂, -CN, -NH-CH₃ and -S-CH₃ and wherein the heteroaryl radical contains 1, 2 or 3 heteroatom(s) independently selected from the group consisting of nitrogen, oxygen and sulfur as ring member(s);
**R¹** represents hydrogen, a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN,-NH-CH₃ and -S-CH₃; or an optionally at least monosubstituted alkyl-aryl radical;
**R³** represents a hydrogen atom; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅,-NO₂, -CN, -NH-CH₃ and -S-CH₃;
**R^{9a}, R^{9b}, R^{9c}, R^{9d}** - if not N(R³)-S(O₂)-A or N-(S(O₂)-A)₂- independently from one another, each represent a hydrogen atom; -NO₂; -NH₂; -SH; -OH; -CN; -C(=O)-H; -C(=O)-R^{'};-C(=O)-O-R^{'}; -OR^{'}; -SR'; -N(R^{'})-S(=O)₂-R^{"}; -NH-R'; -NR^{*}R^{**}; F; Cl, Br; I; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl,-O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, -NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene group and wherein the heteroaryl radical contains 1, 2 or 3 heteroatom(s) independently selected from the group consisting of nitrogen, oxygen and sulfur as ring member(s);
**R¹⁰** represents a hydrogen atom; -NO₂; -NH₂; -SH; -OH; -CN; -C(=O)-OH; -O-R'; -S-R';-C(=O)-OR"; a halogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH_{2:} -C(=O)-NH(C₁₋₅=alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl,-O-C(=O)-C₁₋₅-alkyl, F, Cl, Br, I, -CN, -CF₃, -OCF₃, -SCF₃, -OH, -SH, -NH₂,-NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5-, 6- or 7-membered and may contain 1, 2 or 3 heteroatom(s) as ring member(s) independently selected from the group consisting of nitrogen, oxygen and sulfur;
**R¹²** represents R¹¹,
or
represents 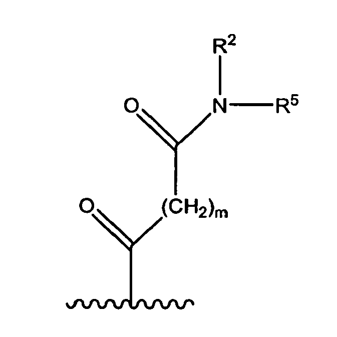 with n being 0;
or represents -C(OC₁₋₄₅-alkyl)-(CH₂)ₘ-R¹¹;
**R¹³** represents a saturated or unsaturated, optionally at least mono-substituted cycloaliphatic radical, or -CHR'R";
**n** being 0, 1, 2, 3 or 4;
**m** being 0, 1, 2, 3 or 4;
**R'** and **R"** identical or different, each represents a saturated or unsaturated, linear or branched, optionally at least mono-substituted aliphatic radical;
**R*** and **R**** identical or different, each represents a saturated or unsatu rated, linear or branched, optionally at least mono-substituted aliphatic radical; or
**R*** and **R**** together with the connecting nitrogen form an optionally at least monosubstituted heterocyclyl radical
**R²** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅,-NO₂, -CN, -NH-CH₃ and -S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅,-NO₂, -CN, -NH-CH₃ and -S-CH₃;
or
**R²** and **R⁵** together with the bridging nitrogen form a saturated, unsaturated or aromatic 3- to 9-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl; -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂,-NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH,-C(=O)-O-C₁₋₅-alkyl, -O-C(=O)-C₁₋₅-alkyl, F, Cl, Br, I, -CN, -CF₃, -OCF₃, -SCF₃, -OH,-SH, -NH₂, -NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

3. Use according to claim 2, wherein the compound binding to the 5HT6-receptor is selected from sulfonamide compound according to formula la, 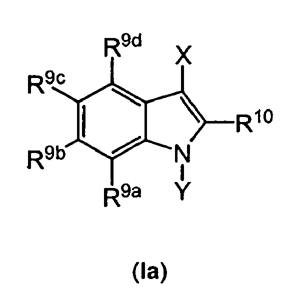 **wherein**
**either**
Y represents S(O₂)-R¹³, while X represents R¹;
**or**
X represents R¹, while Y represents (CH₂)ₙ-R¹¹ or
Y represents R¹, while X represents (CH₂)ₙ-R¹²;
while
one of R^{9a}, R^{9b}, R^{9c}, or R^{9d} represents N(R³)-S(O₂)-A,
**and wherein**
A, R¹; R³, R^{9a}, R^{9b}, R^{9c}, R^{9d}, R¹⁰, R¹¹, R¹², R¹³ and n are as defined in claim 2.

4. Use according to any of claims 2 or 3, wherein the compound binding to the 5HT6-receptor is selected from sulfonamide compounds according to formula I, or la
**wherein**
**--**Z either represents =C(R¹⁰) or -CH₂ or =N;
and wherein
**either**
**Y** represents -S(O₂)-R¹³, while X represents R¹;
or
**X** represents R¹, while **Y** represents (CH₂)ₙ-R¹¹ or
**Y** represents R¹, while **X** represents (CH₂)ₙ-R¹²;
while
one of R^{9a}, R^{9b}, R^{9c}, or R^{9d} represents N(R³)-S(O₂)-A, or N-(S(O₂)-A)₂;
**and wherein**
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;
**R^{9a}, R^{9b}, R^{9c}, R^{9d} -** if not N(R³)-S(O₂)-A or N-(S(O₂)-A)₂- independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl; -O-C₁₋₅-alkyl; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH;
**R¹⁰** represents a hydrogen atom;; a linear or branched optionally at least mono-substituted C₁₋₅-alkyl radical;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
**R¹²** represents **R¹¹,**
or
represents 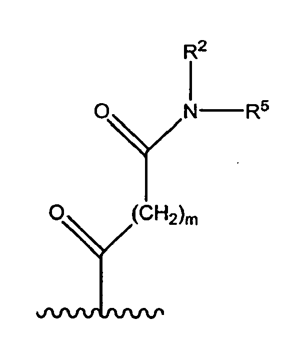 with n being 0;
or represents -C(OC₁₋₅-alkyl)-(CH₂)ₘ-R¹¹;
**R¹³** represents a saturated or unsaturated, optionally at least mono-substituted C₅₋₇-cycloaliphatic radical, or -CHR'R"; with **R'** and **R"** identical or different, each representing a saturated or unsaturated, linear or branched, optionally at least mono-substituted C₁₋₅-alkyl radical;
**n** being 0, 1, 2, 3 or 4;
**m** being 0, 1, 2, 3 or 4;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur.

5. Use according to claims 2 to 4, wherein the compound binding to the 5HT6-receptor is selected from sulfonamide compounds according to formula Ib, or Ic, 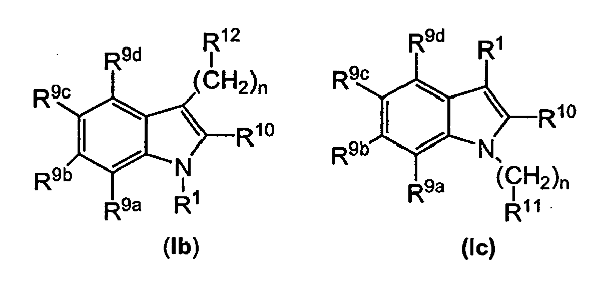 and wherein R¹, R^{9a}, R^{9b}, R^{9c}, R^{9d}, R¹⁰, R¹¹, R¹² and n have the meaning given in claims 2 or 4.

6. Use according to claim 5, wherein the compound binding to the 5HT6-receptor selected from sulfonamide compounds according to formula Ib is a compound according to formulas Iba, Ibb or Ibc, 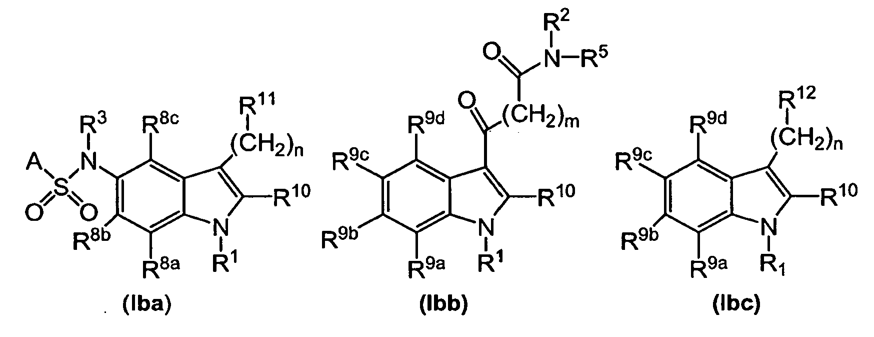 and wherein A, R¹, R², R³, R⁵, R^{9a}, R^{9b}, R^{9c}, R^{9d}, R¹⁰, R¹¹, R¹², m and n have the meaning given in claims 2 or 4 and
wherein
**R^{8a}, R^{8b}, R^{8c}** independently from one another, each represent a hydrogen atom; -NO₂;-NH₂; -SH; -OH; -CN; -C(=O)-H; -C(=O)-R^{'}; -C(=O)-O-R'; -OR^{'}; -SR^{'}; -N(R^{'})-S(=O)₂-R^{"};-NH-R^{'}; -NR^{*}R^{**}; F; Cl, Br; I; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅,-NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, -NH₂,-NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl,-NO₂, -CHO, -CF₂H, -CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene group and wherein the heteroaryl radical contains 1, 2 or 3 heteroatom(s) independently selected from the group consisting of nitrogen, oxygen and sulfur as ring member(s);
preferably **R^{8a}, R^{8b}, R^{8c}** independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl;-O-C₁₋₅-alkyl^{'}; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH.

7. Use according to claim 5, wherein the compound binding to the 5HT6-receptor selected from sulfonamide compounds according to formula Ic is a compound according to formulas Ica, Icb Icc, or Icd, 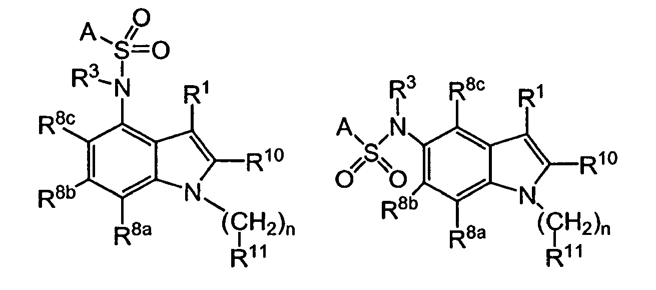 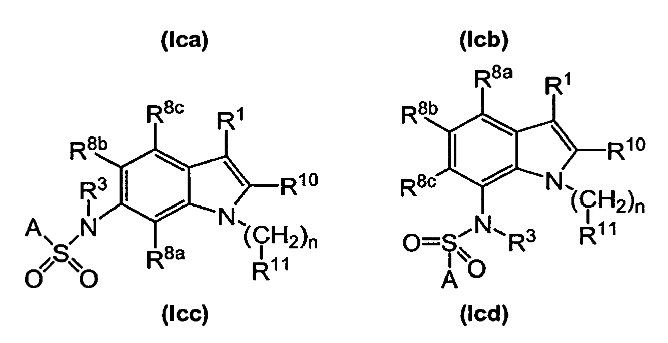 and wherein A, R¹, R³, R¹⁰, R¹¹, R¹², m and n have the meaning given in claims 2 or 4 and wherein R^{8a}, R^{8b}, R^{8c} have the meaning given in claim 6.

8. Use according to claim 6, wherein the compound binding to the 5HT6-receptor is selected from sulfonamide compounds according to formula Iba 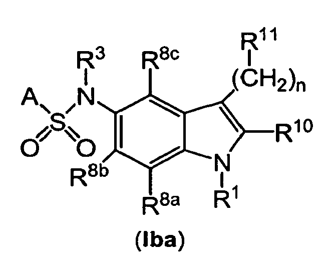 wherein
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;
**R^{8a}, R^{8b}, R^{8c}** each represent a hydrogen atom;
**R¹⁰** represents a hydrogen atom;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
n being 0, 1, 2, 3 or 4;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -ON, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulphur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

9. Use according to claim 8, wherein the compound binding to the 5HT6-receptor is selected from the following group of sulfonamide compounds according to formula Iba consisting of:
[1] N-[3-(2-diethylaminoethyl)-1H-indol-5-yl]-5-chloro-3-methylbenzo[b]thiophene-2-sulphonamide.
[2] N-[3-(2-diethylaminoethyl)-1H-indol-5-yl]naphthalene-1-sulphonamide.
[3] Hydrochloride N-[3-(2-diethylaminoethyl)-1*H*-indol-5-yl]naphthalene-1-sulphonamide.
[4] N-[3-(2-diethylaminoethyl)-1*H*-indol-5-yl]-3,5-dichlorobenzenesulphonamide.
[5] N-[3-(2-diethylaminoethyl)-1*H*-indol-5-yl]-4-phenylbenzenesulphonamide.
[6] N-[3-(2-diethylaminoethyl)-1*H*-indol-5-yl]-5-chlorothiophene-2-sulphonamide.
[7] N-[3-(2-dimethylaminoethyl)-1*H*-indol-5-yl]-5-chloro-3-methylbenzo[b]thiophene-2-sulphonamide.
[8] N-[3-(2-dimethylaminoethyl)-1*H*-indol-5-yl]naphthalene-l-suiphonamide.
[9] N-[3-(2-dimethylamino-ethyl)-1*H*-indol-5-yl]-6-chloroimidazo[2,1-b]thiazol-5-sulphonamide.
[10] N-[3-(1-methylpiperidin-4-yl)-1*H*-indol-5-yl]-5-chloro-3-methylbenzo[b]thiophene-2-sulphonamide.
[11] N-[3-(1-methylpiperidin-4-yl)-1*H*-indol-5-yl]-5-chloro-3-methylbenzo[b]thiophene-2-sulphonamide hydrochloride.
[12] N-[3-(1-methylpiperidin-4-yl)-1*H*-indol-5-yl]naphthalene-1-sulphonamide.
[13] N-[3-(1-methylpiperidin-4-yl)-1*H*-indol-5-yl]naphthalene-1-sulphonamide hydrochloride.
[14] N-[3-(1-methylpiperidin-4-yl)-1*H*-indol-5-yl]-5-chlorothiophene-2-sulphonamide.
[15] N-[3-(1-methylpiperidin-4-yl)-1*H*-indol-5-yl]-4-phenylbenzenesulphonamide.
[16] N-[3-(1-methylpiperidin-4-yl)-1*H*-indol-5-yl]quinoline-8-sulphonamide.
[17] N-[3-(2-diethylaminoethyl)-1*H*-indol-5-yl]naphthalene-2-sulphonamide.
[18] N-[3-(1-methyl-1,2,3,6-tetrahydropyridin-4-yl)-1*H*-indol-5-yl]naphthalene-1-sulphonamide.
[19] N-[3-(4-methylpiperazin-1-yl)methyl-1*H*-indol-5-yl]-5-chloro-3-methylbenzo[b]thiophene-2-sulphonamide.
[20] N-[3-(2-dimethylaminoethyl)-1*H*-indol-5-yl]-5-(2-pyridil)thiophene-2-sulphonamide.
[21] N-[3-(2-dimethylaminoethyl)-1*H*-indol-5-yl]-2,1,3-benzothiadiazol-4-sulphonamide.
[22] N-[3-(2-dimethylaminoethyl)-1*H*-indol-5-yl]quinoline-8-sulphonamide.
[23] N-[3-(2-dimethylaminoethyl)-1*H*-indol-5-yl]-5-chloronaphthalene-2-sulphonamide.
[24] N-[3-(2-dimethylaminoethyl)-1*H*-indol-5-yl]-4-phenoxybenzenesulphonamide.
[25] N-[3-(2-dimethylaminoethyl)-1*H*-indol-5-yl]-4-phenylbenzenesulphonamide.
[26] N-[3-(2-diethylaminoethyl)-1*H*-indol-5-yl]-N-ethyl-naphthalene-2-sulphonamide.
[27] N-{3-[2-(morpholin-4-yl)ethyl]-1H-indol-5-yl}-5-chloro-3-methylbenzo[b]thiophene-2-sulphonamide.
[28] N-{3-[2-(morpholin-4-yl)ethyl]-1H-indol-5-yl}naphthalene-1-sulphonamide.
[29] N-[3-(2-diethylaminoethyl)-1H-indol-5-yl]naphthalene-2-sulphonamide.
[30] N-[3-dimethylaminomethyl-1 H-indol-5-yl]-5-chloro-3-methylbenzo[b]thiophene-2-sulphonamide.
[31] N-[3-(2-dipropylaminoethyl)-1H-indol-5-yl]naphthalene-1-sulphonamide.
[32] N-[3-(2-dipropylaminoethyl)-1H-indol-5-yl]-5-chloro-3-methylbenzo[b]thiophene-2-sulphonamide.
[33] N-[3-(2-dibutylaminoethyl)-1H-indol-5-yl]-5-chloro-3-methylbenzo[b]thiophene-2-sulphonamide.
[34] N-[3-(2-dibutylaminoethyl)-1H-indol-5-yl]naphthalene-1-sulphonamide.
[35] N-[3-(2-diethylaminoethyl)-1H-indol-5-yl]-5-chloronaphthalene-1-sulphonamide.
[36] N-[3-(2-diethylaminoethyl)-1H-indol-5-yl]-trans-β-styrenesulphonamide.
[37] N-[3-(4-methylpiperazin-1-yl)methyl-1*H*-indol-5-yl]-trans-β-styrenesulphonamide.
[38] N-[3-(octahydroindolizin-7-yl)-1H-indol-5-yl]-5-chloro-3-methylbenzo[b]thiophene-2-sulphonamide.
[39] N-[3-(2-diethylaminoethyl)-1*H*-indol-5-yl]-6-chloroimidazo[2,1-b]thiazol-5-sulphonamide.
[40] N-{3-[2-(morpholin-4-yl)ethyl]-1H-indol-5-yl}naphthalene-2-sulphonamide.
[41] N-[3-(4-methylpiperazin-1-yl)methyl-1*H*-indol-5-yl]-α-toluenesulphonamide
[42] N-[3-(3-diethylaminopropyl)-1H-indol-5-yl]naphthalene-2-sulphonamide.
[43] N-[3-(3-diethylaminopropyl)-1H-indol-5-yl]-5-chloro-3-methylbenzo[b]thiophene-2-sulphonamide.
[44] N-{3-[2-(pyrrolidin-1-yl)ethyl]-1H-indol-5-yl}-5-chloro-3-methylbenzo[b]thiophene-2-sulphonamide.
[45] N-{3-[2-(pyrrolidin-1-yl)ethyl]-1H-indol-5-yl}naphthalene-1-sulphonamide.
[46] N-{3-[2-(pyrrolidin-1-yl)ethyl]-1H-indol-5-yl}naphthalene-2-sulphonamide.
[47] N-[3-(2-dipropylaminoethyl)-1H-indol-5-yl]naphthalene-2-sulphonamide.
[48] N-[3-(2-dimethylaminoethyl)-1H-indol-5-yl]-5-chloronaphthalene-1-sulphonamide.
[49] N-[3-(2-dimethylaminoethyl)-1H-indol-5-yl]naphthalene-2-sulphonamide.
[50] N-{3-[2-(morpholin-4-yl)ethyl]-1H-indol-5-yl}quinoline-8-sulphonamide.
[51] N-{3-[2-(morpholin-4-yl)ethyl]-1H-indol-5-yl}-4-phenylbenzenesulphonamide.
[52] N-[3-(4-methylpiperazin-1-yl)ethyl-1*H*-indol-5-yl]naphthalene-2-sulphonamide.
[53] N-[3-(4-methylpiperazin-1-yl)ethyl-1*H*-indol-5-yl]-5-chloronaphthalene-1-sulphonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

10. Use according to claim 6, wherein the compound binding to the 5HT6-receptor is selected from sulfonamide compounds according to formula Ibb 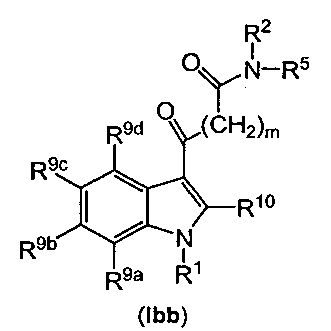 wherein one of **R^{9a}, R^{9b}, R^{9c},** or **R^{9d}** represents N(R³)-S(O₂)-A, or N-(S(O₂)-A)₂;
A represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;
**R^{9a}, R^{9b}, R^{9c}, R^{9d} -** if not N(R³)-S(O₂)-A or N-(S(O₂)-A)₂- independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl; -O-C₁₋₅-alkyl^{'}; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH;
**R¹⁰** represents a hydrogen atom; a linear or branched optionally at least mono-substituted C₁₋₅-alkyl radical;
m represents 0, 1, 2, 3 or 4;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulphur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

11. Use according to claim 10, wherein the compound binding to the 5HT6-receptor is selected from the following group of sulfonamide compounds according to formula Ibb consisting of:
2-[5-(5-Chloro-3-methyl-benzo[b]thiophene-2-sulfonylamino)-1H-indol-3-yl]-N,N-diethyl-2-oxoacetamide.
N,N-Diethyl-2-[5-(naphthalene-2-sulfonylamino)-1H-indol-3-yl]-2-oxo-acetamide.
N,N-Diethyl-2-[5-(naphtalene-1-sulfonylamino)-1H-indol-3-yl]-2-oxo-acetamide. 2-[5-(Biphenyl-4-sulfonylamino)-1H-indol-3-yl]-N,N-diethyl-2-oxo-acetamide.
N,N-Diethyl-2-oxo-2-[5-(quinoline-8-sulfonylamino)-1H-indol-3-yl]-acetamide.
N,N-Dimethyl-2-[5-(naphthalene-2-sulfonylamino)-1H-indol-3-yl]-2-oxo-acetamide.
N,N-Dimethyl-2-[5-(naphtalene-1-sulfonylamino)-1H-indol-3-yl]-2-oxo-acetamide.
2-[5-(5-Chloro-3-methyl-benzo[b]thiophene-2-sulfonyl-amino)-1H-indol-3-yl]-N,N-dimethyl-2-oxo-acetamide.
2-[5-(6-Chloro-imidazo[2,1-b]thiazole-5-sulfonylamino)-1H-indol-3-yl]-N,N-diethyl-2-oxo-acetamide.
2-[5-(6-Chloro-imidazo[2,1-b]thiazole-5-sulfonylamino)-1H-indol-3-yl]-N,N-dimethyl-2-oxo-acetamide.
N,N-Dimethyl-2-[4-(naphthalene-1-sulfonylamino)-1H-indol-3-yl]-2-oxo-acetamide.
2-[4-(5-Chloro-3-methyl-benzo[b]thiophene-2-sulfonylamino)-1H-indol-3-yl]-N,N-dimethyl-2-oxo-acetamide.
2-[4-(6-Chloro-imidazo[2,1-b]thiazole-5-sulfonylamino)-1H-indol-3-yl]-N,N-dimethyl-2-oxo-acetamide.
N,N-Dimethyl-2-[5-[(4-fluoro-3-methyl-phenyl)-1-sulfonylamino]-1H-indol-3-yl]-2-oxo-acetamide.
5-(3-Dimethylaminooxalyl-1H-indol-5-ylsulfamoyl)-3-methyl-benzofuran-2-carboxylic acid ethyl ester.
2-[5-(Biphenyl-4-sulfonylamino)-1H-indol-3-yl]-N,N-dimethyl-2-oxo-acetamide.
N,N-Dimethyl-2-oxo-2-[5-(2-oxo-2,3-dihydro-benzoxazole-6-sulfonylamino)-1H-indol-3-yl]-acetamide.
N,N-Dimethyl-2-oxo-2-[5-(2-oxo-2,3-dihydrobenzo[d]thiazole-6-sulfonamido)-1H-indol-3-yl]acetamide.
2-[5-[(4-Cyclohexyl-phenyl)-1-sulfonylamino]-1H-indol-3-yl]-N,N-dimethyl-2-oxo-acetamide.
N,N-Dimethyl-2-[5-[(4-phenoxy-phenyl)-1-sulfonylamino]-1H-indol-3-yl]-2-oxo-acetamide.
2-(5-(5-chloro-3-methylbenzo[b]thiophene-2-sulfonamido)-2-methyl-1H-indol-3-yl)-N,N-dimethyl-2-oxoacetamide.
2-(5-(6-chloroimidazo[2,1-b]thiazole-5-sulfonamido)-2-methyl-1H-indol-3-yl)-N,N-dimethyl-2-oxoacetamide.
2-(6-(5-chloro-3-methylbenzo[b]thiophene-2-sulfonamido)-1H-indol-3-yl)-N,N-dimethyl-2-oxoacetamide.
N,N-dimethyl-2-(6-(naphthalene-3-sulfonamido)-1H-indol-3-yl)-2-oxoacetamide.
2-(6-(biphenyl-4-sulfonamido)-1H-indol-3-yl)-N,N-dimethyl-2-oxoacetamide.
N,N-dimethyl-2-(6-(naphthalene-1-sulfonamido)-1H-indol-3-yl)-2-oxoacetamide.
N,N-dimethyl-2-(6-(2-(naphthalen-1-yl)ethylsulfonamido)-1H-indol-3-yl)-2-oxoacetamide.
N,N-dimethyl-2-oxo-2-(6-(4-phenoxyphenylsulfonamido)-1H-indol-3-yl)acetamide.
2-(6-(3,4-dichlorothiophene-2-sulfonamido)-1H-indol-3-yl)-N,N-dimethyl-2-oxoacetamide.
2-(6-(3,5-dichlorophenylsulfonamido)-1H-indol-3-yl)-N,N-dimethyl-2-oxoacetamide.
2-(6-(1-chloronaphthalene-6-sulfonamido)-1H-indol-3-yl)-N,N-dimethyl-2-oxoacetamide.
2-(6-(6-chloroimidazo[2,1-b]thiazole-5-sulfonamido)-1H-indol-3-yl)-N,N-dimethyl-2-oxoacetamide.
N,N-diethyl-2-(2-methyl-5-(5-methyl-1-phenyl-1H-pyrazole-4-sulfonamido)-1H-indol-3-yl)-2-oxoacetamide.
N,N-diethyl-2-(2-methyl-5-(1,3,5-trimethyl-1H-pyrazole-4-sulfonamido)-1H-indol-3-yl)-2-oxoacetamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

12. Use according to claim 6, wherein the compound binding to the 5HT6-receptor is selected from sulfonamide compounds according to formula Ibc 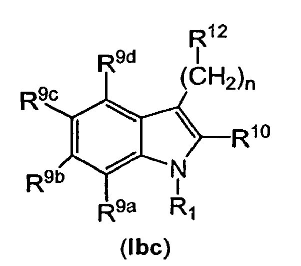 wherein
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branched₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents hydrogen;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ - if present - represents hydrogen;
**R^{9a}, R^{9b}, R^{9c}, R^{9d}-** if not N(R³)-S(O₂)-A or N-(S(O₂)-A)₂ - independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl; -O-C₁₋₅-alkyl^{'}; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; with the proviso that one of **R^{9a}, R^{9b}, R^{9c},** or **R^{9d}** represents N(R³)-S(O₂)-A, or N-(S(O₂)-A)₂;
**R¹⁰** represents a hydrogen atom; a linear or branched optionally at least mono-substituted C₁₋₅-alkyl radical; preferably R¹⁰ represents hydrogen;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
**R¹²** represents R¹¹, or represents -C(OC₁₋₅-alkyl)-(CH₂)ₘ-R¹¹;
**n** being 0, 1, 2, 3 or 4;
**m** being 0, 1, 2, or 3;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R**² and **R**⁵ together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH, - NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

13. Use according to claim 12, wherein the compound binding to the 5HT6-receptor is selected from the following group of sulfonamide compounds according to formula Ibc consisting of:
• 5-chloro-N-(3-(2-(diethylamino)ethyl)-1H-indol-6-yl)-3-methylbenzo[b]thiophene-2-sulfonamide
• N-(3-(2-(diethylamino)ethyl)-1H-indol-6-yl)naphthalene-2-sulfonamide
• N-(3-(2-(diethylamino)ethyl)-1H-indol-6-yl)naphthalene-1-sulfonamide
• 6-chloro-N-(3-(2-(diethylamino)ethyl)-1H-indol-6-yl)imidazo[2,1-b]thiazole-5-sulfonamide
• N-(3-(2-(diethylamino)ethyl)-1H-indol-6-yl)-4-phenylbenzenesulfonamide
• N-(3-(2-(diethylamino)ethyl)-1H-indol-6-yl)-4-phenoxybenzenesulfonamide
• 3,5-dichloro-N-(3-(2-(diethylamino)ethyl)-1H-indol-6-yl)benzenesulfonamide
• 4,5-dichloro-N-(3-(2-(diethylamino)ethyl)-1H-indol-6-yl)thiophene-2-sulfonamide
• 5-chloro-N-(3-(2-(diethylamino)ethyl)-1H-indol-6-yl)naphthalene-1-sulfonamide
• 5-chloro-N-(3-(2-(dimethylamino)ethyl)-1H-indol-6-yl)-3-methylbenzo[b]thiophene-2-sulfonamide
• N-(3-(2-(dimethylamino)ethyl)-1H-indol-6-yi)naphthalene-2-sulfonamide
• N -(3-(2-(dimethylamino)ethyl)-1H-indol-6-yl)naphthalene-1-sulfonamide
• 6-chloro-N-(3-(2-(dimethylamino)ethyl)-1H-indol-6-yl)imidazo[2,1-b]thiazole-5-sulfonamide
• N-(3-(2-(dimethylamino)ethyl)-1H-indol-6-yl)-4-phenylbenzenesulfonamide
• N-(3-(2-(dimethylamino)ethyl)-1H-indol-6-yl)-2-(naphthalen-1-yl)ethanesulfonamide
• N-(3-(2-(dimethylamino)ethyl)-1H-indol-6-yl)-4-phenoxybenzenesulfonamide
• 3,5-dichloro-N-(3-(2-(dimethylamino)ethyl)-1H-indol-6-yl)benzenesulfonamide
• 4,5-dichloro-N-(3-(2-(dimethylamino)ethyl)-1H-indol-6-yl)thiophene-2-sulfonamide
• 5-chloro-N-(3-(2-(dimethylamino)ethyl)-1H-indol-6-yl)naphthalene-1-sulfonamideo
• 5-chloro-N-(3-(2-(dimethylamino)-1-ethoxyethyl)-1H-indol-7-yl)-3-methylbenzo[b]thiophene-2-sulfonamide
• 5-chloro-N-(3-(2-(dimethylamino)ethyl)-1H-indol-7-yl)-3-methylbenzo[b]thiophene-2-sulfonamide
• 7-bis(5-chloro-3-methylbenzo[b]thiophene-2-sulfonyl)amino-3-(2-(diethylamino)-1-ethoxyethyl)-1H-indole
• 5-chloro-N-(3-(2-(diethylamino)-1-ethoxyethyl)-1H-indol-7-yl)-3-methylbenzo[b]thiophene-2-sulfonamide
• 7-bis(5-chloro-3-methylbenzo[b]thiophene-2-sulfonyl)amino-3-(2-(dimethylamino)ethyl)-1H-indole
• 7-bis(5-chloro-3-methylbenzo[b]thiophene-2-sulfonyl)amino-3-(2-(diethylamino)ethyl)-1 H-indole
• 5-chloro-N-(3-(2-(diethylamino)ethyl)-1H-indol-7-yl)-3-methylbenzo[b]thiophene-2-sulfonamide
• 7-bis(6-chloroimidazo[2,1-b]thiazol-5-ylsulfonyl)amino-3-(2-(dimethylamino)ethyl)-1H-indole
• N-(3-(2-(dimethylamino)ethyl)-1H-indol-4-yl)-4-biphenylsulfonamide
• N-(3-(2-(dimethylamino)ethyl)-1H-indol-4-yl)-4-phenoxybenzenesulfonamide
• 3,5-dichloro-N-(3-(2-(dimethylamino)ethyl)-1H-indol-4-yl)benzenesulfonamide
• 5-chloro-N-(3-(2-(dimethylamino)ethyl)-1H-indol-4-yl)-3-methylbenzo[b]thiophene-2-sulfonamide
• N-(3-(2-(dimethylamino)ethyl)-1H-indol-4-yl)naphthalene-1-sulfonamide
• 5-chloro-N-(3-(2-(dimethylamino)ethyl)-1H-indol-4-yl)naphthalene-2-sulfonamide
• N-(3-(2-(dimethylamino)ethyl)-1H-indol-4-yl)naphthalene-2-sulfonamide
• 6-chloro-N-(3-(2-(dimethylamino)ethyl)-1H-indol-4-yl)imidazo[2,1-b]thiazole-5-sulfonamide
• N-(3-(2-(dimethylamino)ethyl)-1H-indol-4-yl)-2-(naphthalen-1-yl)ethanesulfonamide
• 6-bis(6-chloroimidazo[2,1-b]thiazol-5-ylsulfonyl)amino-3-(2-(dimethylamino)ethyl)-1H-indole
• 6-bis(3,5-dichlorobenzenesulfonyl)amino-3-(2-(dimethylamino)ethyl)-1H-indole
• 6-bis(4,5-dichlorothiophene-2-sulfonyl)amino-3-(2-(dimethylamino)ethyl)-1H-indole
• 6-bis(5-chloro-3-methylbenzo[b]thiophene-2-sulfonyl)amino-3-(2-(dimethylamino) -1-ethoxyethyl)-1H-indole
• N-(3-(2-(diethylamino)ethyl)-7-methoxy-1H-indol-5-yl)naphthalene-2-sulfonemide
• N-(3-(2-(diethylamino)ethyl)-7-methoxy-1H-indol-5-yl)benzo[c][1,2,5]thiadiazole-4-sulfonamide
• 6-chloro-N-(3-(2-(diethylamino)ethyl)-7-methoxy-1H-indol-5-yl)imidazo[2,1-b]thiazole-5-sulfonamide
• ethyl 6-(5-chloro-3-methylbenzo[b]thiophene-2-sulfonamido)-3-(1-methylpiperidin-4-yl)-1 H-indole-5-carboxylate
• N-(5-bromo-3-(2-(pyrrolidin-1-yl)ethyl)-1H-indol-7-yl)-6-chloroimidazo[2,1-b]thiazole-5-sulfonamide
• N-(4-bromo-3-(1-methylpiperidin-4-yl)-1H-indol-6-yl)naphthalene-1-sulfonamide
• N-(7-bromo-3-(2-(dimethylamino)ethyl)-1H-indol-5-yl)benzofuran-2-sulfonamide
• N-(7-methoxy-3-(2-(pyrrolidin-1-yl)ethyl)-1H-indol-5-yl)benzo[c][1,2,5]thiadiazole-4-sulfonamide
• N-(7-methoxy-3-(2-(pyrrolidin-1-yl)ethyl)-1H-indol-5-yl)naphthalene-2-sulfonamide
• 6-chloro-N-(7-methoxy-3-(2-(pyrrolidin-1-yl)ethyl)-1H-indol-5-yl)imidazo[2,1-b]thiazole-5-sulfonamide;
optionally in form of one of its stereoisomers, preferably enantiomers or diastereomers, its racemate or in form of a mixture of at least two of its stereoisomers, preferably enantiomers or diastereomers, in any mixing ratio, or a salt,
preferably a physiologically acceptable salt thereof, or a corresponding solvate, respectively;
preferably is selected from:
N-(3-(2-(diethylamino)ethyl)-7-methoxy-1 H-indol-5-yl)naphthalene-2-sulfonamide,
N-(3-(2-(diethylamino)ethyl)-7-methoxy-1 H-indol-5-yl)benzo[c][1,2,5]thiadiazole-4-sulfonamide,
6-chloro-N-(3-(2-(diethylamino)ethyl)-7-methoxy-1H-indol-5-yl)imidazo[2,1-b]thiazole-5-sulfonamide,
ethyl 6-(5-chloro-3-methylbenzo[b]thiophene-2-sulfonamido)-3-(1-methylpiperidin-4-yl)-1 H-indole-5-carboxylate,
N-(5-bromo-3-(2-(pyrrolidin-1-yl)ethyl)-1H-indol-7-yl)-6-chloroimidazo[2,1-b]thiazole-5-sulfonamide,
N-(4-bromo-3-(1-methylpiperidin-4-yl)-1H-indol-6-yl)naphthalene-1-sulfonamide,
N-(7-bromo-3-(2-(dimethylamino)ethyl)-1H-indol-5-yl)benzofuran-2-sulfonamide,
N-(7-methoxy-3-(2-(pyrrolidin-1-yl)ethyl)-1H-indol-5-yl)benzo[c][1,2,5]thiadiazole-4-sulfonamide,
N-(7-methoxy-3-(2-(pyrrolidin-1-yl)ethyl)-1H-indol-5-yl)naphthalene-2-sulfonamide and
6-chloro-N-(7-methoxy-3-(2-(pyrrolidin-1-yl)ethyl)-1H-indol-5-yl)imidazo[2,1-b]thiazole-5-sulfonamide.

14. Use according to claim 7, wherein the compound binding to the 5HT6-receptor is selected from sulfonamide compounds according to formula Ica 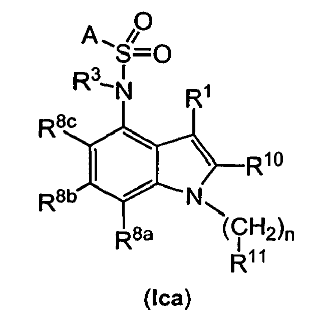 wherein
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
**R^{8a}, R^{8b}, R^{8c}** independently from one another, each represent a hydrogen atom; -NO₂;-NH₂; -SH; -OH; -CN; -C(=O)-H; -C(=O)-O-C₁₋₅-alkyl; -C(=O)-C₁₋₅-alkyl; -O-C₁₋₅-alkyl';-S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R**^{8a}, **R**^{8b}, **R**^{8c} each represent a hydrogen atom;
**R¹⁰** represents a hydrogen atom; a linear or branched optionally at least mono-substituted C₁₋₅-alkyl radical; preferably R¹⁰ represents a hydrogen atom;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
**n** being 0, 1, 2, 3 or 4; preferably n being 2;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

15. Use according to claim 14, wherein the compound binding to the 5HT6-receptor is selected from the following group of sulfonamide compounds according to formula Ica consisting of:
[1] N-[1-(2-dimethylaminoethyl)-1H-indole-4-yl]-5-chloro-3-methylbenzo[b]thiophene-2-sulfonamide,
[2] N-[1-(2-dimethylaminoethyl)-1H-indole-4-yl]-naphtalene-2-sulfonamide,
[3] N-[1-(2-dimethylaminoethyl)-1H-indole-4-yl]-naphtalene-1-sulfonamide,
[4] N-[1-(2-dimethylaminoethyl)-1H-indole-4-yl]-4-phenylbenzenesulfonamide,
[5] N-[1-(2-dimethylaminoethyl)-1H-indole-4-yl]-2-(naphtalene-1-yl)-ethanesulfonamide,
[6] N-[1-(2-dimethylaminoethyl)-1H-indole-4-yl]-4-phenoxybenzenesulfonamide,
[7] N-[1-(2-dimethylaminoethyl)-1H-indole-4-yl]-3,5-dichlorobenzenesulfonamide and
[8] 6-chloro-N-[1-(2-dimethylaminoethyl)-1H-indol-4-yl]-imidazo[2,1-b]thiazole-5-sulfonamide
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

16. Use according to claim 7, wherein the compound binding to the 5HT6-receptor is selected from sulfonamide compounds according to formula Icb 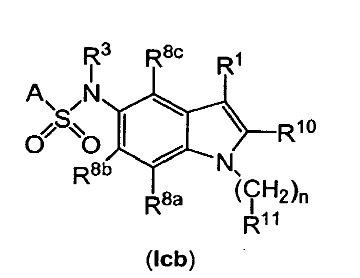 wherein
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
**R^{8a}, R^{8b}, R^{8c}** independently from one another, each represent a hydrogen atom; -NO₂;-NH₂; -SH; -OH; -CN; -C(=O)-H; -C(=O)-O-C₁₋₅-alkyl; -C(=O)-C₁₋₅-alkyl; -O-C₁₋₅-alkyl;-S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{8a}, R^{8b}, R^{8c}** each represent a hydrogen atom;
**R¹⁰** represents a hydrogen atom; a linear or branched optionally at least mono-substituted C₁₋₅-alkyl radical; preferably R¹⁰ represents a hydrogen atom or methyl;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
**n** being 0, 1, 2, 3 or 4; preferably n being 2;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

17. Use according to claim 16, wherein the compound binding to the 5HT6-receptor is selected from the following group of sulfonamide compounds according to formula Icb consisting of:
[1] N-[1-(2-dimethylaminoethyl)-1H-indole-5-yl]-5-chloro-3-methylbenzo[b]thiophene-2-sulfonamide,
[2] N-[1-(2-dimethylaminoethyl)-1H-indole-5-yl]-naphthalene-2-sulfonamide,
[3] N-[1-(2-dimethylaminoethyl)-1H-indole-5-yl]-naphthalene-1-sulfonamide,
[4] N-[1-(2-dimethylaminoethyl)-1H-indole-5-yl]-5-chloronaphthalene-1-sulfonamide,
[5] N-[1-(2-dimethylaminoethyl)-1H-indole-5-yl]-benzenesulfonamide,
[6] N-[1-(2-dimethylaminoethyl)-1H-indole-5-yl]-quinoline-8-sulfonamide,
[7] N-[1-(2-dimethylaminoethyl)-1H-indole-5-yl]-4-phenoxybenzenesulfonamide,
[8] N-[1-(2-dimethylaminoethyl)-1H-indole-5-yl]-4-methylbenzenesulfonamide,
[9] N-[1-(2-dimethylaminoethyl)-1H-indole-5-yl]-5-chlorothiophene-2-sulfonamide,
[10] N-[1-(2-dimethylaminoethyl)-1H-indole-5-yl]-benzo[1,2,5]thiadiazole-4-sulfonamide,
[11] N-[1-(2-dimethylaminoethyl)-1H-indole-5-yl]-6-chloroimidazo[2,1-b]thiazole-5-sulfonamide,
[12] N-[1-(2-dimethylaminoethyl)-1H-indole-5-yl]-3,5-dichlorobenzenesulfonamide,
[13] N-[1-(2-dimethylaminoethyl)-1H-indole-5-yl]-3-bromobenzenesulfonamide,
[14] N-[1-(2-dimethylaminoethyl)-1H-indole-5-yl]-3-nitrobenzenesulfonamide,
[15] N-[1-(2-dimethylaminoethyl)-1H-indole-5-yl]-1-phenylmethanesulfonamide,
[16] N-[1-(2-pyrrolidine-1-yl-ethyl)-1H-indole-5-yl]-naphthalene-2-sulfonamide,
[17] N-[1-(2-pyrrolidine-1-yl-ethyl)-1H-indole-5-yl]-naphthalene-1-sulfonamide,
[18] N-[1-(2-pyrrolidine-1-yl-ethyl)-1H-indole-5-yl]-5-chloro-3-methylbenzo[b]thiophene-2-sulfonamide,
[19] trans-N-[1-(2-dimethylaminoethyl)-1H-indole-5-yl]-2-phenylethenesulfonamide,
[20] N-[1-(2-dimethylaminoethyl)-1H-indole-5-yl]-4,5-dichlorothiophene-2-sulfonamide,
[21] N-[1-(2-dimethylaminoethyl)-1H-indole-5-yl]-4-acetylbenzenesulfonamide,
[22] N-[1-(2-dimethylaminoethyl)-1H-indole-5-yl]-4-bromobenzenesulfonamide,
[23] N-[1-(2-dimethylaminoethyl)-1H-indole-5-yl]-4-methoxybenzenesulfonamide,
[24] N-[3-(2-diethylaminoethyl)-1H-indole-5-yl]-5-chloro-3-methylbenzo[b]thiophene-2-sulfonamide,
[25] N-[1-(2-dimethylaminoethyl)-1H-indole-5-yl]-4-nitrobenzenesulfonamide,
[26] N-[1-(2-dimethylaminoethyl)-1H-indole-5-yl]-4-fluorobenzenesulfonamide,
[27] N-[1-(2-diethylaminoethyl)-1H-indole-5-yl]-6-chloroimidazo[2,1-b]thiazole-5-sulfonamide,
[28] N-[1-(2-pyrrolidine-1-yl-ethyl)-1H-indole-5-yl]- ]-6-chloroimidazo[2,1-b]thiazole-5-sulfonamide,
[29] N-(1-(2-(diethylamino)ethyl)-1H-indol-5-yl)-naphthalene-2-sulfonamide,
[30] N-(1-(2-(diethylamino)ethyl)-1H-indol-5-yl)-naphthalene-1-sulfonamide,
[31] N-(1-(2-(diethylamino)ethyl)-1H-indol-5-yl)-4-phenylbenzenesulfonamide,
[32] 5-chloro-N-(1-(2-(dimethylamino)ethyl)-2-methyl-1H-indol-5-yl)-3-methylbenzo[b]thiophene-2-sulfonamide,
[33] N-(1-(2-(dimethylamino)ethyl)-2-methyl-1H-indol-5-yl)-naphthalene-2-sulfonamide,
[34] N-(1-(2-(dimethylamino)ethyl)-2-methyl-1H-indol-5-yl)-naphthalene-1-sulfonamide,
[35] 6-chloro-N-(1-(2-(dimethylamino)ethyl)-2-methyl-1H-indol-5-yl)imidazo[2,1-b]thiazole-5-sulfonamide,
[36] N-(1-(2-(dimethylamino)ethyl)-2-methyl-1H-indol-5-yl)-4-phenylbenzenesulfonamide,
[37] N-(1-(2-dimethylamino)ethyl)-2-methyl-1H-indol-5-yl)-2-(naphth-1-yl)-ethanesulfonamide,
[38] N-(1-(2-(dimethylamino)ethyl)-2-methyl-1H-indol-5-yl)-4-phenoxy-benzenesulfonamide,
[39] 3,5-dichloro-N-(1-(2-dimethylamino)ethyl)-2-methyl-1H-indol-5-yl)-benzenesulfonamide,
[40] N-(1-(2-(dimethylamino)ethyl)-2-methyl-1H-indol-5-yl)benzo[b]thiophene-3-sulfonamide,
[41] N-(1-(2-(diethylamino)ethyl)-1H-indol-5-yl)benzo[b]thiophene-3-sulfonamide and
[42] N-(1-(2-(dimethylamino)ethyl)-1H-indol-5-yl)benzo[b]thiophene-3-sulfonamide,
[43] 5-chloro-3-methyl-N-(1-(3-(piperidin-1-yl)propyl)-1H-indol-5-yl)benzo[b]thiophene-2-sulfonamide,
[44] N-(1-(3-(piperidin-1-yl)propyl)-1H-indol-5-yl)naphthalene-2-sulfonamide,
[45] N-(1-(3-(piperidin-1-yl)propyl)-1H-indol-5-yl)naphthalene-1-sulfonamide,
[46] 6-chloro-N-(1-(3-piperidin-1-yl)propyl)-1H-indol-5-yl)imidazo[2,1-b]thiazole-5-sulfonamide,
[47] 4-phenyl-N-(1-(3-(piperidin-1-yl)propyl)-1H-indol-5-yl)benzenesulfonamide,
[48] 2-(naphth-1-yl)-N-(1-(3-(piperidin-1-yl)propyl)-1H-indol-5-yl)ethanesulfonamide,
[49] 4-phenoxy-N-(1-(3-(piperidin-1-yl)propyl)-1H-indol-5-yl)benzenesulfonamide,
[50] 3,5-dichloro-N-(1-(3-(piperidin-1-yl)propyl)-1H-indol-5-yl)benzenesulfonylamide,
[51] 4,5-dichloro-N-(1-(3-(piperidin-1-yl)propyl)-1H-indol-5-yl)thiophene-2-sulfonamide and
[52] 5-chloro-N-(1-(3-(piperidin-1-yl)propyl)-1H-indol-5-yl)naphthalene-1-sulfonamide,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

18. Use according to claim 7, wherein the compound binding to the 5HT6-receptor is selected from sulfonamide compounds according to formula Icc 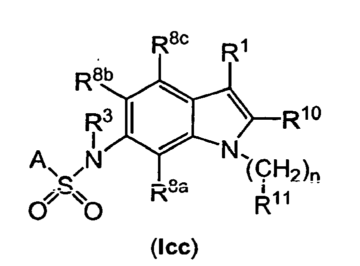 wherein
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
**R^{8a}, R^{8b}, R^{8c}** independently from one another, each represent a hydrogen atom; -NO₂;-NH₂; -SH; -OH; -CN; -C(=O)-H; -C(=O)-O-C₁₋₅-alkyl; -C(=O)-C₁₋₅-alkyl; -O-C₁₋₅-alkyl;-S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{8a}, R^{8b}, R^{8c}** each represent a hydrogen atom;
**R¹⁰** represents a hydrogen atom; a linear or branched optionally at least mono-substituted C₁₋₅-alkyl radical; preferably R¹⁰ represents a hydrogen atom;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
**n** being 0, 1, 2, 3 or 4; preferably n being 2;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulphur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

19. Use according to claim 18, wherein the compound binding to the 5HT6-receptor is selected from the following group of sulfonamide compounds according to formula Icc consisting of:
[1] N-[1-(2-Dimethylaminoethyl)-1H-indol-6-yl]-5-chloro-3-methylbenzo[b]thiophene-2-sulfonamide,
[2] N-[1-(2-Dimethylaminoethyl)-1H-indol-6-yl]-naphthalene-2-sulfonamide,
[3] N-[1-(2-Dimethylaminoethyl)-1H-indol-6-yl]-naphthalene-1-sulfonamide,
[4] N-[1-(2-Dimethylaminoethyl)-1H-indol-6-yl]-6-chlorolmidazo[2,1-b]thiazole-5-sulfonamide,
[5] N-[1-(2-Dimethylaminoethyl)-1H-indol-6-yl]-4-phenylbenzenesulfonamide,
[6] N-[1-(2-Dimethylaminoethyl)-1H-indol-6-yl]-2-(naphthalene-1-yl)-ethanesulfonamide,
[7] N-[1-(2-Dimethylaminoethyl)-1H-indol-6-yl]-4-phenoxybenzenesulfonamide,
[8] N-[1-(2-Dimethylaminoethyl)-1H-indol-6-yl]-3.5-dichlorobenzenesulfonamide,
[9] 5-Chloro-3-methyl-N-[1-[2-(pyrrolidin-1-yl)ethyl-1H-indol-6-yl]-benzo[b]thiophene-2-sulfonamide,
[10] N-(1-[2-(Pyrrolidin-1-yl)ethyl]-1H-indol-6-yl]-napthalene-2-sulfonamide,
[11] N-[1-[2-Pyrrolidin-1-yl]ethyl]-1H-indol-6-yl]-naphthalene-1-sulfonamide,
[12] 6-Chloro-N-[1-[2-(pyrrolidin-1-yl)ethyl]-1H-indol-6-yl]-imidazo[2,1-b]thiazole-5-sulfonamide,
[13] 4-Phenyl-N-(1-(2-(pyrrolidin-1-yl)ethyl)-1H-indol-6-yl)-benzenesulfonamide
[14] 2-(Naphthyl-1-yl)-N-(1-(2-(pyrrolidin-1-yl)ethyl)-1H-indol-6-yl)-ethansulfonamide,
[15] 4-Phenoxy-N-(1-(2-(pyrrolidin-1-yl)ethyl)-1H-indol-6-yl)-benzenesulfonamide and
[16] 3,5-Dichloro-N-(1-(2-(pyrrolidin-1-yl)-1H-indol-6-yl)-benzenesulfonamide,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

20. Use according to claim 7, wherein the compound binding to the 5HT6-receptor is selected from sulfonamide compounds according to formula Icd 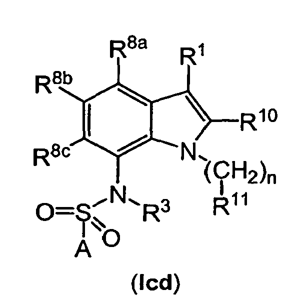 wherein
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
**R^{8a}, R^{8b}, R^{8c}** independently from one another, each represent a hydrogen atom; -NO₂;-NH₂; -SH; -OH; -CN; -C(=O)-H; -C(=O)-O-C₁₋₅-alkyl; -C(=O)-C₁₋₅-alkyl; -O-C₁₋₅-alkyl';-S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{8a}, R^{8b}, R^{8c}** each represent a hydrogen atom;
**R¹⁰** represents a hydrogen atom; a linear or branched optionally at least mono-substituted C₁₋₅-alkyl radical; preferably R¹⁰ represents a hydrogen atom;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl. -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6-or 7-membered and may contain 1, 2 or 3 heteroatom(s) as ring member(s) independently selected from the group consisting of nitrogen, oxygen and sulfur;
n being 0, 1, 2, 3 or 4; preferably n being 2;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulphur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

21. Use according to claim 20, wherein the compound binding to the 5HT6-receptor is selected from the following group of sulfonamide compounds according to formula Icd consisting of:
[1] N-[1-(2-dimethylaminoethyl)-1H-indole-7-yl]-naphtalene-1-sulfonamide,
[2] N-[1-(2-dimethylaminoethyl)-1H-indole-7-yl]-5-chloro-3-methylbenzo[b]thiophene-2-sulfonamide,
[3] N-[1-(2-dimethylaminoethyl)-1H-indole-7-yl]-4-phenylbenzenesulfonamide and
[4] N-[1-(2-dimethylaminoethyl)-1H-indole-7-yl]-6-chloroimidazo[2,1-b]thiazole-5-sulfonamide
[5] 5-chloro-3-methyl-N-(1-(2-(pyrrolidin-1-yl)ethyl)-1H-indol-7-yl)-benzo[b]thiophen-2-sulfonamide,
[6] N-(1-(2-(pyrrolidin-1-yl)ethyl)-1H-indol-7-yl)naphthalene-1-sulfonamide,
[7] 6-chloro-N-(1-(2-(pyrroldin-1-yl)ethyl)-1H-indol-7-yl)imidazo[2,1-b]thiazole-5-sulfonamide and
[8] 2-(naphth-1-yl)-N-(1-(2-(pyrrolidin-1-yl)ethyl)-1H-indol-7-yl)ethansulfonamide
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

22. Use according to any of claims 2 to 4, wherein the compound binding to the 5HT6-receptor is selected from sulfonamide compounds according to formula Id 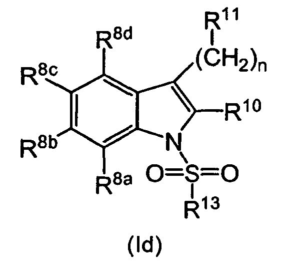 wherein
**R^{8a}, R^{8b}, R^{8c}, R^{8d}** independently from one another, each represent a hydrogen atom;-NO₂; -NH₂; -SH; -OH; -CN; -C(=O)-H; -C(=O)-O-C₁₋₅-alkyl; -C(=O)-C₁₋₅-alkyl; -O-C₁₋₅-alkyl; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH;
**R¹⁰** represents a hydrogen atom;; a linear or branched optionally at least mono- substituted C₁₋₅-alkyl radical; preferably R2 represents H;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
**n** being 0, 1, 2, 3 or 4;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅- alkyl, -O-C₁₋₅-alkyl, -S-C₁₋₅-alkyl, oxo (=O), thioxo (=S), F, Cl, Br, I, -CN, -CF₃,-OCF₃, - -OH, -SH, -NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃, -SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively

23. Use according to claim 22, wherein the compound binding to the 5HT6-receptor is selected from the following group of sulfonamide compounds according to formula Id consisting of:
[1] 1-Cyclohexanesulfonyl-3-(1-methyl-1,2,3,6-tetrahydropyridine-4-yl)-5-nitro-1H-indole,
[2] 5-Chloro-1-cyclohexanesulfonyl-3-(1-methyl-1,2,3,6-tetrahydropyridine-4-yl)-1H-indole,
[3] 5-Amino-1-cyclohexanesulfonyl-3-(1-methyl-1,2,3,6-tetrahydropyridine-4-yl)-1H-indole and
[4] 1-Cyclohexanesulfonyl-5-fluoro-3-(1,2,3,5,8,8a-hexahydro-indolizine-7-yl)-1H-indole hydrochloride;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

24. Use according to claim 2, wherein the compound binding to the 5HT6-receptor is selected from sulfonamide compounds according to formula le 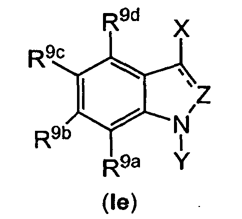 wherein
**wherein**
**--Z** either represents -CH₂ or =N;
**and wherein**
**X** represents R¹, while **Y** represents (CH₂)ₙ-R¹¹ or
**Y** represents R¹, while **X** represents (CH₂)ₙ-R¹¹;
while
one of R^{9a} , R^{9b}, R^{9c}, or R^{9d} represents N(R³)-S(O₂)-A;
**and wherein**
**A** represents a 5- to 14-membered aryl, alkyl-aryl, heterocyclyl or alkyl-heterocyclyl radical, which may be substituted with 1, 2 or 3 substituent(s) independently selected from the group consisting of -CF₃, C₁₋₅-alkyl, -O-C₁₋₅-alkyl, -C(=O)-OH, - C(=O)-C₁₋₅-alkyl, C(=O)-O-C₁₋₅-alkyl, oxo (=O), F, Cl, Br, I, -CN, -OCF₃, , -OH, -SH, -NH₂, -NH(C₁₋₅-alkyl), -N(C₁₋₅-alkyl)₂, -NO₂, -CHO, -CF₂H, -CFH₂, cyclopropyl, cyclobutyl, cyclopentyl, cyclohexyl, phenyl, phenoxy, benzyl, and pyridinyl;
**R¹** represents hydrogen, a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or benzyl; preferably R¹ represents a hydrogen atom;
**R³** represents a hydrogen atom; a linear or branchedC₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃; preferably R³ represents a hydrogen atom;
**R^{9a}, R^{9b}, R^{9c}, R^{9d}** - if not N(R³)-S(O₂)-A - independently from one another, each represent a hydrogen atom; -NO₂; -NH₂; -SH; -OH; -CN; -C(=O)-H; -C(=O)-O-C₁₋₅-alkyl; -C(=O)-C₁₋₅-alkyl; -O-C₁₋₅-alkyl; -S-C₁₋₅-alkyl; - F; Cl, Br; I; a linear or branched C₁₋₅ alkyl radical which may be substituted with 1, 2 or 3 substituent(s) independently selected from the group consisting of F, Cl, Br, -OH, -NH₂, and -SH; preferably **R^{9a}, R^{9b}, R^{9c}, R^{9d} -** if not N(R³)-S(O₂)-A - each represent a hydrogen atom;
**R¹¹** represents NR²R⁵ or a saturated or unsaturated 3-, 4-, 5-, 6-, 7- or 8-membered cycloaliphatic radical, which may be substituted with 1, 2 or 3 substituent(s) independently selected from the group consisting of C₁₋₅-alkyl, -O-C₁₋₅-alkyl, F, Cl, Br, I, -CF₃, -OCF₃, -OH, -NH₂, -CF₂H, -CFH₂, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - F, Cl, Br, I, -CN, -CF₃, -OCF₃, -OH, -SH, -NH₂, - -CF₂H, -CFH₂, and whereby the rings of the ring system are 5-, 6- or 7-membered and may contain 1, 2 or 3 heteroatom(s) as ring member(s) independently selected from the group consisting of nitrogen, oxygen and sulfur;
**n** being 0, 1, 2, 3 or 4;
**R²** represents a hydrogen atom; or a linear or branched C₁₋₅ alkyl radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and-S-CH₃;; optionally at least monosubstituted alkyl-aryl; or C(O)-R with R being an optionally at least mono-substituted aryl,
**R⁵** represents a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, and -O-C₂H₅;
or
**R²** and **R⁵** together with the bridging nitrogen form a saturated, unsaturated or aromatic 5- to 7-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), F, Cl, Br, I, -CN, -CF₃, -OCF₃, - -OH, -SH,-NH₂, - -CF₂H, -CFH₂, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅-alkyl, -O-C₁₋₅-alkyl, F, Cl, Br, I, -CN, -CF₃, -OCF₃,-SCF₃, -OH, -SH, -NH₂, -CF₂H, -CFH₂, and whereby the rings of the ring system are 5-, 6- or 7-membered and may contain 1, 2 or 3 heteroatom(s) independently selected from the group consisting of nitrogen, oxygen and sulfur;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

25. Use according to claim 24, wherein the compound binding to the 5HT6-receptor is selected from the following group of sulfonamide compounds according to formula le consisting of:
[1] N-(1-(2-(Dimethylamino)ethyl)-1H-indazol-6-yl)napthalene-2-sulphonamide;
[2] 5-Chloro-N-(1-(2-(dimethylamino)ethyl)-1H-indazol-6-yl)-3-methylbenzo[b]thiophene-2-sulfonamide;
[3] Naphthalene-2-sulfonic acid [3-(1-methyl-piperidin-4-yl)-1H-indazol-5-yl]-amide,
[4] 5-Chloro-3-methyl-benzo[b]thiophene-2-sulfonic acid [3-(1-methyl-piperidin-4-yl)-1H-indazol-5-yl]-amide,
[5] Naphthalene-1-sulfonic acid [3-(1-methyl-piperidin-4-yl)-1H-indazol-5-yl]-amide,
[6] 4-Phenylbenzene-4-sulfonic acid [3-(1-methyl-piperidin-4-yl)-1H-indazol-5-yl]-amide,
[7] N-[3-(1-Methyl-piperidin-4-yl)-1H-indazol-5-yl]-4-phenoxy-benzenesulfonamide
[8] N-[3-(1-Methyl-piperidin-4-yl)-1H-indazol-5-yl]-benzenesulfonamide;
[9] N-[1-(2-Dimethylamino)ethyl)-2,3-dihydro-1H-indol-6-yl]-6-chloro-imidazo[2,1-b]thiazol-5-sulfonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

26. Use according to claim 1 wherein the medicament is for the treatment of weight-gain induced by a therapeutic drug.

27. Use according to claim 1 wherein the medicament is for the treatment of obesity induced by a therapeutic drug.

28. Use according to claim 1 wherein the medicament is for the treatment of cardio-metabolic risk factors induced by a therapeutic drug.

29. Use according to claim 1 wherein the therapeutic drug is selected rom the group consisting of:
antipsychotics, comprising the atypical antipsychotics and typical neuroleptics, antidepressants including MAOls, TCAs, SSRls, "third generation" and
atypical antidepressants, anti-manic agents, corticosteroids, agents for insulin resistance, impaired glucose tolerance and Type 2 diabetes, antihypertensives, anti-gastric ulceratives, anti-epileptics, drugs used in medicaments for neuropathic pain, antihistamines, drugs used in medicaments for migraine, benzodiazepines, contraceptives and drugs used in hormone replacement therapy.

30. Use according to claim 1 wherein the therapeutic drug is an antipsychotic, comprising the atypical antipsychotics and typical neuroleptics, antidepressants including MAOls, TCAs, SSRls, "third generation" and atypical antidepressants, anti-manic agents.
